# EUROPEAN PATENT APPLICATION

(11) **EP 4 806 918 A1**
(43) Date of publication of application: **16.09.2026**
(21) Application number: 24887975.1
(22) Date of filing: 06.11.2024
(51) Int. Cl.: C07K 16/28, A61K 39/395, A61K 45/06, A61P 31/00

(54) **FULLY HUMAN ANTIBODY TARGETING HUMAN PD-L1**

(30) Priority: 06.11.2023 CN 202311466254
(71) Applicant: Nanjing Probio Biotech Co., Ltd., Nanjing, Jiangsu 211100 (CN)
(72) Inventor: LI, Yingyu, Nanjing, Jiangsu 211100 (CN); XIN, Yang, Nanjing, Jiangsu 211100 (CN); LIN, Qianwen, Nanjing, Jiangsu 211100 (CN); CHEN, Li, Nanjing, Jiangsu 211100 (CN); LIANG, Yu, Nanjing, Jiangsu 211100 (CN)
(74) Representative: Ström & Gulliksson AB
(86) International application number: PCT/CN2024/130101
(87) International publication number: WO 2025/098364

(57) **Abstract**

A fully human antibody targeting human PD-L1, comprising a fully human antagonistic PD-L1 antibody or an antigen-binding portion thereof. The present invention further relates to a nucleic acid molecule encoding the antibody or the antigen-binding portion thereof, an expression vector for expressing the antibody or the antigen-binding portion thereof, a host cell and a method, and a treatment method using the antibody or the antigen-binding portion thereof, the nucleic acid molecule, the expression vector and/or the host cell.

## Description

### CROSS-REFERENCE TO RELATED APPLICATIONS

The present application claims priority to Chinese Patent Application No. CN 202311466254.8, filed on November 06, 2023, which is incorporated herein by reference in its entirety.

### TECHNICAL FIELD

The present application relates to a fully human antagonistic antibody targeting human PD-L1.

### BACKGROUND

Carcinogenesis results from the loss of normal regulatory functions of normal somatic cells and the accumulation of gene mutations to a certain extent. The immune system can launch an effective attack only after recognizing cancer cells.

Tumor-associated antigens (TAAs), which are cell surface proteins highly or characteristically expressed in tumor cells, represent one of the breakthrough points for the immune system to recognize cancer cells. Targeted therapy targeting TAAs constitutes one of the important approaches in current cancer treatment. Antibodies targeting TAAs can guide NK cells or T cells to tumor cells with high TAA expression, thereby specifically killing the tumor cells. Currently, several cancer immunotherapeutic drugs relying on this mechanism are already available on the market, such as rituximab and trastuzumab, which have achieved good clinical efficacy.

However, targeted therapy is not effective in all cancer patients. Studies have shown that cancer cells have developed numerous mechanisms to evade immune surveillance. For example, cancer cells may disguise themselves as normal somatic cells or transmit inhibitory signals to surrounding immune cells. In particular, cancer cells highly express PD-L1, which binds to PD-1 on the surface of immune cells, leading to dysfunction and exhaustion of the immune cells.

PD-1 is a membrane receptor protein mainly expressed on the surface of immune cells, such as T cells and B cells. PD-L1 is one of the ligands for PD-1, and is a type I transmembrane protein with a molecular weight of 40 KDa. Normal human proteins express PD-L1, which binds to PD-1 on immune cells, thereby reducing the immune response against normal human cells and maintaining a certain level of self-tolerance. As described above, solid tumors and hematological malignancies often upregulate the expression of PD-L1 and exploit the PD-1/PD-L1 pathway to suppress immune cell function, thereby evading immune surveillance. PD-L1 can also be expressed on immune cells within the tumor microenvironment (TME), including tumor-associated macrophages (TAMs), myeloid-derived suppressor cells (MDSCs), dendritic cells (DCs), T cells, B cells, macrophages, bone marrow-derived mast cells, as well as on some non-immune cells, making the TME tumor-permissive. Studies have shown that the PD-1/PD-L1 pathway reduces the activity of CD8⁺ T lymphocytes and CD4⁺ T lymphocytes and inhibits the proliferation thereof, thereby inhibiting the function of T lymphocytes in the TME and weakening the immune killing effect against tumors.

PD-1 and PD-L1 inhibitors have been widely used in tumor immunotherapy. By blocking the binding between PD-1 and PD-L1, the immune suppression is relieved, and the attack of immune cells on tumor cells is activated, thereby achieving antitumor effects. These immunotherapeutic drugs have demonstrated significant efficacy in the treatment of various malignant tumors, including melanoma, non-small cell lung cancer, renal cell carcinoma, bladder cancer, head and neck squamous cell carcinoma, etc., and have been widely applied in clinical practice.

### SUMMARY

By using phage display technology, the inventors of the present application have screened several fully human anti-PD-L1 antibodies. The fully human antibodies have amino acid sequences that are 100% derived from humans, offering the advantages of lower immunogenicity and better safety.

The PD-L1 antibody of the present application, as compared to prior art antibodies such as Avelumab or Tecentriq, has comparable or higher PD-1 binding affinity, comparable or higher PD-L1-PD-1 blocking activity, comparable or higher safety, and/or comparable or higher *in vivo* antitumor efficacy.

Therefore, in a first aspect, the present application relates to an isolated monoclonal antibody (e.g., a chimeric or human antibody) or an antigen-binding portion thereof that is capable of specifically binding to PD-L1 (e.g., human, monkey, or mouse PD-L1), which may comprise: i) a heavy chain variable region, which may comprise VH CDR1, VH CDR2, and VH CDR3, wherein VH CDR1, VH CDR2, and VH CDR3 may comprise amino acid sequences of: (1) GRTALTYA (SEQ ID NO: 1), INWSGSMT (SEQ ID NO: 2), and AATRTAITMPPQRDGVDY (SEQ ID NO: 3) respectively; or (2) GSSFSLAV (SEQ ID NO: 8), ISSAAGT (SEQ ID NO: 9), and WRGAVPGDPYGR (SEQ ID NO: 10) respectively; and/or ii) a light chain variable region, which may comprise VL CDR1, VL CDR2, and VL CDR3, wherein VL CDR1, VL CDR2, and VL CDR3 may comprise amino acid sequences of: (1) QDISNY (SEQ ID NO: 4), DAS, and QQYDNLPRT (SEQ ID NO: 5), respectively; (2) QSVSNNY (SEQ ID NO: 11), GAS, and QQYGNSPGT (SEQ ID NO: 12), respectively; (3) QSLVHSDGNTY (SEQ ID NO: 15), EVS, and MQGTHWPWT (SEQ ID NO: 16), respectively. Further provided is a variant of the antibody or the antigen-binding portion thereof described above, which comprise up to about 3 amino acid residue substitutions, e.g., 1, 2, or 3 amino acid residue substitutions, in each CDR, as compared to the antibody or the antigen-binding portion thereof described above.

The isolated monoclonal antibody or the antigen-binding portion thereof of the present application may comprise a heavy chain variable region and a light chain variable region, wherein VH CDR1, VH CDR2, VH CDR3, VL CDR1, VL CDR2 and VL CDR3 may comprise amino acid sequences of: (1) GRTALTYA (SEQ ID NO: 1), INWSGSMT (SEQ ID NO: 2), AATRTAITMPPQRDGVDY (SEQ ID NO: 3), QDISNY (SEQ ID NO: 4), DAS, and QQYDNLPRT (SEQ ID NO: 5), respectively; (2) GSSFSLAV (SEQ ID NO: 8), ISSAAGT (SEQ ID NO: 9), WRGAVPGDPYGR (SEQ ID NO: 10), QSVSNNY (SEQ ID NO: 11), GAS, and QQYGNSPGT (SEQ ID NO: 12), respectively; or (3) GSSFSLAV (SEQ ID NO: 8), ISSAAGT (SEQ ID NO: 9), WRGAVPGDPYGR (SEQ ID NO: 10), QSLVHSDGNTY (SEQ ID NO: 15), EVS, and MQGTHWPWT (SEQ ID NO: 16), respectively. In some embodiments, VH CDR1, VH CDR2, VH CDR3, VL CDR1, VL CDR2, and VL CDR3 may comprise amino acid sequences of GRTALTYA (SEQ ID NO: 1), INWSGSMT (SEQ ID NO: 2), AATRTAITMPPQRDGVDY (SEQ ID NO: 3), QDISNY (SEQ ID NO: 4), DAS, and QQYDNLPRT (SEQ ID NO: 5), respectively. Further provided is a variant of the antibody or the antigen-binding portion thereof described above, which comprise up to about 3 amino acid residue substitutions, e.g., 1, 2, or 3 amino acid residue substitutions, in each CDR, as compared to the antibody or the antigen-binding portion thereof described above.

The heavy chain variable region of the antibody or the antigen-binding portion thereof of the present application may comprise an amino acid sequence having at least 80%, 85%, 90%, 91%, 92%, 93%, 94%, 95%, 96%, 97%, 98%, 99%, or 100% sequence identity to SEQ ID NO: 6, 13, or 17.

The light chain variable region of the antibody or the antigen-binding portion thereof of the present application may comprise an amino acid sequence having at least 80%, 85%, 90%, 91%, 92%, 93%, 94%, 95%, 96%, 97%, 98%, 99%, or 100% sequence identity to SEQ ID NO: 7, 14, or 18.

The heavy chain variable region and light chain variable region of the antibody or the antigen-binding portion thereof of the present application may comprise amino acid sequences having at least 80%, 85%, 90%, 91%, 92%, 93%, 94%, 95%, 96%, 97%, 98%, 99%, or 100% sequence identity to: (1) SEQ ID NOs: 6 and 7, respectively; (2) SEQ ID NOs: 13 and 14, respectively; or (3) SEQ ID NOs: 17 and 18, respectively.

In some embodiments, the isolated monoclonal antibody or the antigen-binding portion thereof of the present application may comprise a heavy chain variable region and a light chain variable region, wherein VH CDR1, VH CDR2, VH CDR3, VL CDR1, VL CDR2 and VL CDR3 may comprise amino acid sequences of GRTALTYA (SEQ ID NO: 1), INWSGSMT (SEQ ID NO: 2), AATRTAITMPPQRDGVDY (SEQ ID NO: 3), QDISNY (SEQ ID NO: 4), DAS, and QQYDNLPRT (SEQ ID NO: 5), respectively. The heavy chain variable region may comprise an amino acid sequence having at least 80%, 85%, 90%, 91%, 92%, 93%, 94%, 95%, 96%, 97%, 98%, 99%, or 100% sequence identity to SEQ ID NO: 6. The light chain variable region may comprise an amino acid sequence having at least 80%, 85%, 90%, 91%, 92%, 93%, 94%, 95%, 96%, 97%, 98%, 99%, or 100% sequence identity to SEQ ID NO: 7.

In some embodiments, the isolated monoclonal antibody or the antigen-binding portion thereof of the present application may comprise a heavy chain variable region and a light chain variable region, wherein VH CDR1, VH CDR2, VH CDR3, VL CDR1, VL CDR2 and VL CDR3 may comprise amino acid sequences of GSSFSLAV (SEQ ID NO: 8), ISSAAGT (SEQ ID NO: 9), WRGAVPGDPYGR (SEQ ID NO: 10), QSVSNNY (SEQ ID NO: 11), GAS, and QQYGNSPGT (SEQ ID NO: 12), respectively. The heavy chain variable region may comprise an amino acid sequence having at least 80%, 85%, 90%, 91%, 92%, 93%, 94%, 95%, 96%, 97%, 98%, 99%, or 100% sequence identity to SEQ ID NO: 13. The light chain variable region may comprise an amino acid sequence having at least 80%, 85%, 90%, 91%, 92%, 93%, 94%, 95%, 96%, 97%, 98%, 99%, or 100% sequence identity to SEQ ID NO: 14.

The present application further provides an isolated monoclonal antibody or an antigen-binding portion thereof that is capable of specifically binding to PD-L1, which may comprise: i) a heavy chain variable region comprising VH CDR1, VH CDR2 and VH CDR3; and ii) a light chain variable region comprising VL CDR1, VL CDR2 and VL CDR3, wherein VH CDR1, VH CDR2, VH CDR3, VL CDR1, VL CDR2 and VL CDR3 may comprise VH CDR1, VH CDR2, VH CDR3, VL CDR1, VL CDR2, and VL CDR3 comprised in selected heavy and light chain variable regions, and the selected heavy chain variable region and light chain variable region may comprise amino acid sequences set forth in: (1) SEQ ID NOs: 6 and 7, respectively; (2) SEQ ID NOs: 13 and 14, respectively; or (3) SEQ ID NOs: 17 and 18, respectively.

The isolated monoclonal antibody or the antigen-binding portion thereof of the present application may comprise a heavy chain constant region and/or a light chain constant region, wherein the N-terminus of the heavy chain constant region is linked to the C-terminus of the heavy chain variable region, and the N-terminus of the light chain constant region is linked to the C-terminus of the light chain variable region. The heavy chain constant region may be an IgG, IgD, IgA, IgM, or IgE heavy chain constant region, preferably a heavy chain constant region that naturally has, or upon engineering has, binding affinity for FcRs and/or complement system proteins, or a functional fragment thereof, e.g., a fragment comprising the hinge region, CH₂, and CH₃ of the heavy chain constant region. In one embodiment, the heavy chain constant region may be an IgG1 heavy chain constant region, e.g., a human IgG1 heavy chain constant region, which comprises, e.g., an amino acid sequence set forth in Uniprot Accession No. P01857-1. The light chain constant region may be a κ or λ light chain constant region. In some embodiments, the light chain constant region may be a human κ light chain constant region, which comprises, e.g., an amino acid sequence set forth in Uniprot Accession No. P01834.

In some embodiments, the antibody of the present application comprises or consists of two heavy chains and two light chains, wherein each of the heavy chains comprises the heavy chain constant region sequence, heavy chain variable region sequence, and/or CDR sequences described above, and each of the light chains comprises the light chain constant region sequence, light chain variable region sequence, and/or CDR sequences described above. In some embodiments, the antibody or the antigen-binding portion thereof of the present application may be a Fab, an F(ab')₂ fragment, Fv, scFv, (scFv)₂, etc.

The antibody or the antigen-binding portion thereof of the present application may be, e.g., human or chimeric.

The antibody or the antigen-binding portion thereof of the present application may be antagonistic.

The present application further provides an immunoconjugate comprising the antibody or the antigen-binding portion thereof of the present application, wherein the antibody or the antigen-binding portion thereof can be linked to a therapeutic agent, e.g., a cytotoxic molecule or an anti-cancer agent. The present application further provides a bispecific molecule comprising the antibody or the antigen-binding portion thereof of the present application, wherein the antibody or the antigen-binding portion thereof can be linked to a second functional moiety, e.g., a second antibody, and the second functional moiety has a binding specificity different from that of the antibody or the binding portion thereof of the present application. In another aspect, the antibody or the antigen-binding portion thereof of the present application may be part of a chimeric antigen receptor (CAR) or a genetically engineered T cell receptor (TCR). The present application further provides an immune cell comprising the CAR and/or TCR described above, including T cells, NK cells, etc. The antibody or the antigen-binding portion thereof of the present application may also be encoded by or carried by an oncolytic virus

The present application further encompasses a nucleic acid molecule encoding the antibody or the antigen-binding portion thereof, the immunoconjugate, the bispecific molecule, the CAR, or the TCR of the present application. The present application further provides an expression vector and a host cell. The expression vector may comprise the nucleic acid molecule of the present application. The host cell may comprise the expression vector of the present application, or have the nucleic acid molecule of the present application integrated into the genome thereof.

The present application further provides a method for preparing the antibody or the antigen-binding portion thereof, the immunoconjugate, the bispecific molecule, the CAR, or the TCR of the present application using the host cell of the present application, the method comprising: (i) expressing the antibody or the antigen-binding portion thereof, the immunoconjugate, the bispecific molecule, the CAR, or the TCR in the host cell; and (ii) isolating the antibody or the antigen-binding portion thereof, the immunoconjugate, the bispecific molecule, the CAR, or the TCR from the host cell or a culture thereof.

The present application further provides a composition comprising the antibody or the antigen-binding portion thereof, the immunoconjugate, the bispecific molecule, the immune cell, the oncolytic virus, the nucleic acid molecule, the expression vector, or the host cell of the present application. In some embodiments, the composition may be a pharmaceutical composition, which may further comprise a pharmaceutically acceptable carrier.

In another aspect, the present application provides a method for treating or ameliorating a PD-L1-associated disease in a subject, the method comprising administering to the subject a therapeutically effective amount of the pharmaceutical composition of the present application. The PD-L1-associated disease may be a tumor, including solid tumors. The solid tumors include, but are not limited to, melanoma, non-small cell lung cancer, renal cell carcinoma, bladder cancer, or head and neck squamous cell carcinoma. In some embodiments, the pharmaceutical composition of the present application can be administered with at least one other antibody. In another embodiment, the pharmaceutical composition of the present application can be administered with a cytokine (e.g., IL-2 and/or IL-21) or a co-stimulatory antibody (e.g., a CD137 antibody and/or a GITR antibody). In another embodiment, the antibody or the antigen-binding portion thereof of the present application can be administered with a chemotherapeutic agent, which may be a cytotoxic agent.

The present application further provides a method of reversing or alleviating immunosuppression in a subject, the method comprising administering to the subject an effective amount of the pharmaceutical composition of the present application. In some embodiments, the method is used for reversing or alleviating immunosuppression in the tumor microenvironment, the method comprising administering to a tumor site an effective amount of the pharmaceutical composition of the present application.

The present application further provides a method for enhancing an immune response in a subject, the method comprising administering to the subject an effective amount of the pharmaceutical composition of the present application.

The present application further relates to the use of the composition of the present application, in particular a pharmaceutical composition, in the preparation of a drug for treating or alleviating a PD-L1-associated tumor, reversing or alleviating immunosuppression, or enhancing an immune response.

The antibody or the antigen-binding portion thereof of the present application may also be used, for example, for *in-vitro* antigen detection, which comprises contacting a sample to be tested with the antibody or the antigen-binding portion thereof of the present application.

It should be noted that, in the present application, particularly in the claims, terms such as "comprising" and "including" may have the meanings conferred under the Chinese Patent Law. Moreover, terms such as "consisting essentially of" have the meanings conferred under the Chinese Patent Law, for example, permitting the presence of elements not expressly recited, but excluding elements that exist in the prior art or that affect the basic or novel characteristics of the present invention.

Other features and advantages of the present disclosure will become more apparent with reference to the following detailed description and examples, which shall not be construed as limiting. The content of all of the documents, Genbank records, patents, and published patent applications cited in the present application is expressly incorporated herein by reference.

### BRIEF DESCRIPTION OF THE DRAWINGS

The following detailed description is provided by way of example, but is not intended to limit the present invention to the specific embodiments described, and can be better understood with reference to the accompanying drawings.
FIG. 1 shows the binding affinity of the PD-L1 antibodies of the present application for human PD-L1 protein as detected by ELISA;
FIG. 2 shows the binding affinity of the PD-L1 antibodies of the present application for cells expressing human PD-L1 as detected by FACS;
FIG. 3 shows the binding affinity of the PD-L1 antibodies of the present application for cells expressing monkey PD-L1 as detected by FACS;
FIG. 4 shows the binding affinity of the PD-L1 antibodies of the present application for cells expressing mouse PD-L1 as detected by FACS;
FIG. 5 shows the detection results of binding affinity of the PD-L1 antibodies of the present application AHP30005 (A), AHP30019 (B) and AHP31319 (C), as well as Avelumab (D), to human PD-L1 protein;
FIG. 6 shows the inhibitory effect of Avelumab on the binding of the PD-L1 antibodies of the present application to human PD-L1 protein;
FIG. 7 shows the inhibitory effect of Tecentriq on the binding of the PD-L1 antibodies of the present application to human PD-L1 protein; and
FIG. 8 shows the *in-vitro* blocking activity of the PD-L1 antibodies of the present application on the PD-1/PD-L1 interaction.

### DETAILED DESCRIPTION

Unless otherwise indicated, the terms used herein have ordinary meanings as set forth in dictionaries, textbooks and technical references, or as commonly understood by those skilled in the art. The following descriptions of certain terms are provided only for the convenience of understanding the present application and are not intended to impose special limitations on such terms unless otherwise indicated.

As used herein and in the appended claims, the singular forms "a", "an", and "the" include plural referents unless otherwise clearly specified in the context.

The term "or" refers to a single element from the list of selectable elements unless otherwise clearly indicated in the context.

The term "comprise" or "include" refers to the inclusion of the stated elements, integers, or steps, but not to the exclusion of any other elements, integers, or steps. The term "comprise" or "include" used herein, unless indicated, also encompasses combinations of the stated elements, integers, or steps.

The term "PD-L1" refers to programmed death receptor-ligand 1. The term includes variants, homologs, orthologs, and paralogs. For example, an antibody specific for human PD-L1 may, in certain circumstances, cross-react with a PD-L1 protein from another species, e.g., monkey.

The term "human PD-L1" refers to a PD-L1 protein having a human amino acid sequence, e.g., a PD-L1 protein having the amino acid sequence under NCBI accession number NP_001300958.1 (Nasr S et al., (2023) *BMC Cancer* 23(1): 817). The term "monkey PD-L1" refers to a PD-L1 protein having a monkey amino acid sequence, e.g., a PD-L1 protein having the amino acid sequence under NCBI accession number XP_033093166.1.

The term "antibody" herein is intended to include full-length antibodies of IgG, IgA, IgD, IgE, and IgM and any antigen-binding fragment (i.e., antigen-binding portion). A full-length antibody is a glycoprotein comprising at least two heavy (H) chains and two light (L) chains, the heavy chain and the light chain being linked by disulfide bonds. Each heavy chain consists of a heavy chain variable region (abbreviated as V_{H} or VH) and a heavy chain constant region. The heavy chain constant region consists of three domains: C_{H1}, C_{H2}, and C_{H3}. Each light chain consists of a light chain variable region (abbreviated as V_{L} or VL) and a light chain constant region. The light chain constant region consists of one domain, C_{L}. The V_{H} and V_{L} regions may also be divided into hypervariable regions, known as complementarity-determining regions (CDRs), which are separated by more highly conserved framework regions (FRs). Each V_{H} or V_{L} consists of three CDRs and four FRs arranged from the amino terminus to the carboxyl terminus in the order of FR1, CDR1, FR2, CDR2, FR3, CDR3, and FR4. The variable regions of the heavy chains and light chains comprise binding domains that interact with antigens. The constant regions of the antibody can mediate the binding of immunoglobulins to host tissues or factors, including binding to various cells of the immune system (e.g., effector cells) and the first component (C1q) of the classical complement system. A "functional fragment" of an antibody constant region refers to a fragment of the constant region that retains certain desired functions, e.g., a fragment of the heavy chain constant region that retains the binding affinity for FcRs or components of the complement system, such as an Fc fragment.

The term "antigen-binding portion" of an antibody (or abbreviated as an antibody portion) herein refers to one or more fragments of an antibody that retain the ability to specifically bind to an antigen (e.g., PD-L1 protein). It has been demonstrated that the antigen-binding functionality of an antibody can be implemented by fragments of a full-length antibody. Examples of binding fragments comprised in the "antigen-binding portion" of an antibody include: (i) a Fab fragment, a monovalent fragment consisting of V_{L}, V_{H}, C_{L}, and C_{H1}; (ii) an F(ab')₂ fragment, a bivalent fragment comprising two Fab fragments linked by a disulfide bridge in the hinge region; (iii) an Fd fragment consisting of V_{H} and C_{H1}; (iv) an Fv fragment consisting of the V_{L} and V_{H} of a single arm of an antibody; (v) a dAb fragment (Ward et al., (1989) Nature 341: 544-546) consisting of V_{H}; (vi) an isolated complementarity-determining region (CDR); and (vii) a dAb-V_{L}, a fragment comprising a single variable domain and a heavy chain constant domain. Furthermore, although the two domains of the Fv fragment, V_{L} and V_{H}, are encoded by different genes, they can be linked by recombinant methods via a synthetic linker that brings the two into a single protein chain, in which the V_{L} and V_{H} regions are paired to form a monovalent molecule. These single-chain antibodies are also intended to be included within the meaning of the term. These antibody fragments can be obtained by conventional techniques known to those skilled in the art, and the fragments can be functionally screened in the same manner as intact antibodies.

The term "isolated antibody" as used herein refers to an antibody that is substantially free of other antibodies having different antigen specificities. For example, an isolated antibody that specifically binds to a PD-L1 protein is substantially free of antibodies that specifically bind to antigens other than the PD-L1 protein. However, an isolated antibody that specifically binds to a human PD-L1 protein may have cross-reactivity with other antigens, e.g., PD-L1 proteins from other species. Furthermore, the isolated antibody is substantially free of other cellular materials and/or chemical substances.

The term "monoclonal antibody" or "mAb" or "monoclonal antibody composition" refers to an antibody molecule product consisting of a single molecule. The monoclonal antibody composition exhibits single binding specificity and affinity for a specific epitope.

The term "human antibody" or "fully human antibody" refers to an antibody in which the framework regions and CDRs of the variable regions are derived from human germline immunoglobulin sequences. Furthermore, if the antibody comprises a constant region, the constant region is also derived from human germline immunoglobulin sequences. The human antibody of the present application may comprise amino acid residues not encoded by human germline immunoglobulin sequences, e.g., mutations introduced by *in vitro* random or point mutations or by *in vivo* somatic mutations. However, the term "human antibody" or "fully human antibody" does not include antibodies in which CDR sequences derived from other mammalian species are inserted into human framework sequences.

The term "chimeric antibody" refers to an antibody that combines genetic material from one species with genetic material from another species. In particular, a chimeric antibody in the present application refers to an antibody obtained by combining non-human genetic material with human genetic material.

The terms "antibody recognizing an antigen" and "antibody specific for an antigen" are used interchangeably herein with the term "antibody specifically binding to an antigen".

Herein, the terms "specifically recognizes" or "specifically binds to" a target, e.g., human PD-L1, mean that an antibody or an antigen-binding fragment is capable of distinguishing the target biomolecule from one or more reference molecules, and exhibits binding affinity or binding affinity for the target biomolecule that is, for example, 1-fold, 5-fold, 10-fold or higher than that for other reference molecules. Methods for determining specificity include, but are not limited to, SPR, Western blotting, ELISA, RIA, ECL, IRMA assays, and peptide scanning.

The term "not substantially bind" to a protein or cell refers to not binding to the protein or cell or not binding to the protein or cell with high affinity, i.e., the K_{D} for binding to the protein or cell is 1.0 × 10⁻⁶ M or higher, preferably 1.0 × 10⁻⁵ M or higher, more preferably 1.0 × 10⁻⁴ M or higher or 1.0 × 10⁻³ M or higher, and more preferably 1.0 × 10⁻² M or higher.

The term "EC₅₀", also known as half-maximal effective concentration, refers to the concentration of an antibody that induces 50% of the maximal effect.

The term "IC₅₀" refers to half-maximal inhibitory concentration, i.e., the concentration of a drug or inhibitor required to inhibit a specified biological process by half.

The term "subject" includes any human or non-human animal. The term "non-human animal" includes all vertebrates, such as mammals and non-mammals, such as non-human primates, sheep, dogs, cats, cows, horses, chickens, amphibians, and reptiles; although mammals such as non-human primates, sheep, dogs, cats, cows, and horses are preferred.

The term "therapeutically effective amount" refers to an amount of the antibody of the present application sufficient to prevent or reduce symptoms associated with a disease or disorder (e.g., cancer). The therapeutically effective amount is dependent on the disease being treated, and the actual effective amount can be readily determined by those skilled in the art.

The term "sequence identity" herein refers to the percentage of nucleotides/amino acids in a sequence that are identical to the nucleotides/amino acid residues in a reference sequence after a sequence alignment, and if desired, gaps are introduced in the sequence alignment to achieve the maximum percent sequence identity between the two sequences. Those skilled in the art can perform pairwise sequence alignment or multiple sequence alignment using various methods, e.g., computer software, to determine the percent sequence identity between two or more nucleic acid or amino acid sequences. Examples of such computer software include Clustal Omega, T-coffee, Kalign, MAFFT, etc.

The PD-L1 antibody of the present application, as compared to prior art antibodies such as Avelumab or Tecentriq, has: i) comparable or better binding affinity and binding specificity for (human, monkey, or mouse) PD-L1; ii) comparable or higher PD-L1-PD-1 blocking activity; iii) comparable or lower immunogenicity, and thus comparable or higher safety; and/or iv) comparable or higher *in vivo* anti-tumor efficacy.

The preferred antibody of the present application is a monoclonal antibody. Furthermore, the antibody or the antigen-binding portion thereof may be, e.g., human or chimeric.

Exemplary antibodies or antigen-binding portions thereof of the present application are those whose structural and chemical properties are described below.

The sequences or sequence numbers of the heavy chain variable regions and the light chain variable regions of the antibodies or the antigen-binding portions thereof of the present application are listed in Table 1. The CDRs of the heavy chain variable region and the CDRs of the light chain variable region are determined according to the IMGT numbering system, and the CDR sequences or sequence numbers thus determined are listed in Table 1. The CDRs of the heavy chain variable region and the CDRs of the light chain variable region of the antibodies or the antigen-binding portions thereof of the present application may also be determined according to the Chothia, Kabat, AbM, or Contact numbering system based on the full-length variable region sequences.

The antibodies of the present application may have a heavy chain constant region, e.g., a native or engineered one that exhibits binding affinity for FcRs and/or complement system proteins, particularly those with high binding affinity for FcRs and/or complement system proteins. In some embodiments, the heavy chain constant region may be an IgG1 constant region, e.g., a human IgG1 constant region comprising an amino acid sequence set forth in Uniprot Accession No. P01857-1. The light chain constant region may be a κ constant region, e.g., a human κ constant region, which may comprise an amino acid sequence set forth in Uniprot Accession No. P01834.

The V_{H} and/or V_{L} sequences (or CDR sequences) of other PD-L1 antibodies that bind to human PD-L1 can be "mixed and matched" with the V_{H} and/or V_{L} sequences (or CDR sequences) of the antibodies of the present application. Preferably, when V_{H} and V_{L} (or CDRs therein) are mixed and matched, the V_{H} sequence in a specific V_{H}/V_{L} pair can be replaced by a structurally similar V_{H} sequence. Similarly, preferably, the V_{L} sequence in a specific V_{H}/V_{L} pair is replaced by a structurally similar V_{L} sequence.

Therefore, in one embodiment, the antibody or the antigen-binding portion thereof of the present application comprises:
(a) a heavy chain variable region comprising an amino acid sequence listed in Table 1; and
(b) a light chain variable region comprising an amino acid sequence listed in Table 1, or a V_{L} of another PD-L1 antibody, wherein the antibody specifically binds to human PD-L1.

In another embodiment, the antibody or the antigen-binding portion thereof of the present application comprises:
(a) CDR1, CDR2, and CDR3 of the heavy chain variable region listed in Table 1; and
(b) CDR1, CDR2, and CDR3 of the light chain variable region listed in Table 1, or CDRs of another PD-L1 antibody, wherein the antibody specifically binds to human PD-L1.

In another embodiment, the antibody or the antigen-binding portion thereof of the present application comprises CDR2 of the heavy chain variable region of a PD-L1 antibody, and CDRs of other antibodies that bind to human PD-L1, e.g., CDR1 and/or CDR3 of the heavy chain variable region, and/or CDR1, CDR2 and/or CDR3 of the light chain variable region of another PD-L1 antibody.

Furthermore, it is well known in the art that the CDR3 domain, independently of CDR1 and/or CDR2, can alone determine the binding specificity of antibodies for the same antigen, and it can be predicted that multiple antibodies having the same binding specificity can be generated based on the CDR3 sequence. See, e.g., Klimka et al., British J. of Cancer 83(2): 252-260 (2000); Beiboer et al., J. Mol. Biol. 296: 833-849 (2000); Rader et al., Proc. Natl. Acad. Sci. U.S.A. 95: 8910-8915 (1998); Barbas et al., J. Am. Chem. Soc. 116: 2161-2162 (1994); Barbas et al., Proc. Natl. Acad. Sci. U.S.A. 92: 2529-2533 (1995); Ditzel et al., J. Immunol. 157: 739-749 (1996).

In another embodiment, the antibody or the antigen-binding portion thereof of the present application comprises CDR2 of the heavy chain variable region of a PD-L1 antibody, and at least CDR3 of the heavy and/or light chain variable region of a PD-L1 antibody, or CDR3 of the heavy and/or light chain variable region of another PD-L1 antibody, wherein the antibody or the antigen-binding portion thereof is capable of specifically binding to human PD-L1. Preferably, these antibodies or antigen-binding portions thereof (a) compete for binding to PD-L1; (b) retain functional properties; (c) bind to the same epitope; and/or (d) have a binding affinity similar to that of the PD-L1 antibody or the antigen-binding portion thereof of the present application. In another embodiment, the antibody or the antigen-binding portion thereof may further comprise CDR2 of the light chain variable region of the PD-L1 antibody or the antigen-binding portion thereof of the present application, or CDR2 of the light chain variable region of another PD-L1 antibody, wherein the antibody or the antigen-binding portion thereof specifically binds to human PD-L1. In another embodiment, the antibody of the present application may comprise CDR1 of the heavy/light chain variable region of the PD-L1 antibody or the antigen-binding portion thereof of the present application, or CDR1 of the heavy and/or light chain variable region of another PD-L1 antibody, wherein the antibody or the antigen-binding portion thereof specifically binds to human PD-L1.

In another embodiment, the antibody or the antigen-binding portion thereof of the present application comprises heavy and/or light chain variable region sequences, or CDR1, CDR2 and CDR3 sequences, with one or more conservative modifications relative to those of the PD-L1 antibody or the antigen-binding portion thereof of the present application. It is known in the art that some conservative sequence modifications do not eliminate the antigen-binding affinity. See, e.g., Brummell et al., (1993) Biochem 32: 1180-1188.

Therefore, in one embodiment, the antibody or the antigen-binding portion thereof comprises a heavy chain variable region and/or a light chain variable region, wherein the heavy chain variable region and the light chain variable region each comprise CDR1, CDR2, and CDR3, wherein:
(a) CDR1 of the heavy chain variable region comprises a sequence listed in Table 1, or a conservative modification thereof; and/or
(b) CDR2 of the heavy chain variable region comprises a sequence listed in Table 1, or a conservative modification thereof; and/or
(c) CDR3 of the heavy chain variable region comprises a sequence listed in Table 1, or a conservative modification thereof; and/or
(d) CDR1, and/or CDR2, and/or CDR3 of the light chain variable region comprise a sequence listed in Table 1, and/or a conservative modification thereof; and
(e) the antibody or the antigen-binding portion thereof specifically binds to human PD-L1.

The antibody of the present disclosure has one or more of the following functional characteristics, e.g., high affinity and high specific binding to human PD-L1, and high blocking activity for PD-L1-PD-1.

In multiple embodiments, the antibody or the antigen-binding portion thereof may be, e.g., human or chimeric.

The term "conservative sequence modification" as used herein refers to an amino acid modification that does not significantly affect or alter the binding properties of the antibody. Such conservative modifications include amino acid substitutions, additions and deletions. Modifications can be introduced into the antibody of the present application or the antigen-binding portion thereof by standard techniques known in the art, e.g., point mutations and PCR-mediated mutations. Conservative amino acid substitutions are those in which an amino acid residue is substituted with an amino acid residue having a similar side chain. Groups of amino acid residues having similar side chains are known in the art. Such groups of amino acid residues include amino acids with basic side chains (e.g., lysine, arginine, and histidine), amino acids with acidic side chains (e.g., aspartic acid, and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β-branched side chains (e.g., threonine, valine, and isoleucine), and amino acids with aromatic side chains (e.g., tyrosine, phenylalanine, tryptophan, and histidine). Therefore, one or more amino acid residues in the CDR regions of the antibody or the antigen-binding portion thereof of the present application can be substituted with other amino acid residues from the same side chain group, and the resulting antibody can be tested for retained function (i.e., the functions described above) using the functional assays described herein.

The antibody or the antigen-binding portion thereof of the present application can be prepared as a genetically modified antibody using, as a starting material, an antibody having one or more V_{H}/V_{L} sequences of the PD-L1 antibody or the antigen-binding portion thereof of the present application. The antibody can be genetically modified by modifying one or more residues in one or both variable regions (i.e., V_{H} and/or V_{L}) (e.g., in one or more CDR regions and/or one or more framework regions) to improve binding affinity and/or increase similarity to naturally occurring antibodies of certain species. For example, alternatively, the antibody can be genetically modified by modifying residues in the constant region, for example, to alter the effector function of the antibody.

Variable region modifications may involve mutating amino acid residues in the V_{H} and/or V_{L} CDR1, CDR2, and/or CDR3 regions, thereby improving one or more binding properties (e.g., affinity) of the antibody of interest. Point mutations or PCR-mediated mutations can be performed to introduce mutations, and the effects thereof on antibody binding or other functional properties can be assessed in *in vitro* or *in vivo* assays known in the art. Preferably, conservative modifications known in the art are introduced. The mutations may be amino acid substitutions, additions, or deletions, but substitutions are preferred. Furthermore, generally no more than one, two, three, four, or five residues in the CDR regions are altered.

Furthermore, in another embodiment, the present application provides an isolated PD-L1 monoclonal antibody or an antigen-binding portion thereof, comprising a heavy chain variable region and a light chain variable region, which comprise: (a) a V_{H} CDR1 region comprising a sequence of the present application, or an amino acid sequence having one, two, three, four, or five amino acid substitutions, deletions, or additions; (b) a V_{H} CDR2 region comprising a sequence of the present application, or an amino acid sequence having one, two, three, four, or five amino acid substitutions, deletions, or additions; (c) a V_{H} CDR3 region comprising a sequence of the present application, or an amino acid sequence having one, two, three, four, or five amino acid substitutions, deletions, or additions; (d) a V_{L} CDR1 region comprising a sequence of the present application, or an amino acid sequence having one, two, three, four, or five amino acid substitutions, deletions, or additions; (e) a V_{L} CDR2 region comprising a sequence of the present application, or an amino acid sequence having one, two, three, four, or five amino acid substitutions, deletions, or additions; and (f) a V_{L} CDR3 region comprising a sequence of the present application, or an amino acid sequence having one, two, three, four, or five amino acid substitutions, deletions, or additions.

The genetically engineered antibodies of the present application include those in which genetic modifications are made in the framework residues of the V_{H} and/or V_{L} to, for example, alter antibody properties. Framework modifications include mutating one or more residues in the framework regions, or even in one or more CDR regions, to remove T-cell epitopes, thereby reducing potential immunogenicity of the antibody. This method is also referred to as "deimmunization" and is described in more detail in U.S. Pat. Publ. No. 20030153043.

Furthermore, as an alternative to modifications in the framework or CDR regions, the antibodies of the present application can be genetically engineered to include genetic modifications in the Fc region, generally to alter one or more functional properties of the antibody, e.g., serum half-life, complement fixation, Fc receptor binding, and/or antibody-dependent cellular cytotoxicity. Furthermore, the antibodies of the present application can be chemically modified (e.g., one or more chemical functional moieties can be attached to the antibody) or modified to alter their glycosylation, so as to change one or more functional properties of the antibody.

In one embodiment, the hinge region of C_{H1} is modified to alter, e.g., to increase or decrease, the number of cysteine residues in the hinge region. This method is further described in U.S. Pat. No. 5,677,425. Cysteine residues in the C_{H1} hinge region are altered, for example, to facilitate the assembly of the heavy chain and light chain or to increase/decrease stability of the antibody.

In another embodiment, the Fc-hinge region of the antibody is mutated to increase or decrease the biological half-life of the antibody. More specifically, one or more amino acid mutations are introduced into the C_{H2}-C_{H3} linking region of the Fc-hinge fragment such that the antibody has reduced SpA binding affinity relative to the SpA binding of a natural Fc-hinge domain. This method is described in more detail in U.S. Pat. No. 6,165,745.

In another embodiment, the glycosylation of the antibody is modified. For example, deglycosylated antibodies (i.e., antibodies lacking glycosylation) can be prepared. Glycosylation can be altered, for example, to increase the affinity of the antibody for an antigen. Such glycosylation modifications can be achieved, for example, by altering one or more glycosylation sites in the sequence of the antibody. For example, one or more amino acid substitutions can be made to eliminate one or more framework glycosylation sites in the variable regions, thereby eliminating glycosylation at these sites. Such deglycosylation may increase the affinity of the antibody for the antigen. See, e.g., U.S. Pat. Nos. 5,714,350 and 6,350,861.

Furthermore, antibodies with altered glycosylation patterns can be prepared, for example, hypofucosylated antibodies with reduced levels of fucose residues, or antibodies with increased bisecting GlcNac structures. The altered glycosylation patterns have been demonstrated to increase the ADCC activity of the antibodies. Such glycosylation modifications can be performed, for example, by expressing the antibody in host cells with an altered glycosylation system. Cells with altered glycosylation systems are known in the art, including but not limited to Slc35c1 gene knockout cell lines, FUT8 knockout cell lines, the variant CHO cell line Lec13, the rat hybridoma cell line YB2/0, cell lines comprising small interfering RNAs specifically directed against the FUT8 gene, and cell lines co-expressing β-1,4-N-acetylglucosaminyltransferase III and Golgi α-mannosidase II. These cells can be used as host cells for expressing the recombinant antibodies of the present application to prepare antibodies with altered glycosylation.

Another modification of the antibodies herein is PEGylation. The antibodies can be PEGylated, for example, to increase the biological (e.g., serum) half-life of the antibodies. To PEGylate an antibody, the antibody or the fragment thereof is generally reacted with polyethylene glycol (PEG), such as a reactive ester or aldehyde derivative of PEG, under conditions such that one or more PEG groups are attached to the antibody or antibody fragment. Preferably, PEGylation is performed by an acylation reaction or an alkylation reaction with a reactive PEG molecule (or a similar reactive water-soluble polymer). The term "polyethylene glycol" as used herein includes any form of PEG used to derivatize other proteins, such as mono(C₁-C₁₀)alkoxy- or aryloxy-polyethylene glycol or polyethylene glycol maleimide. In certain embodiments, the antibody to be PEGylated is a deglycosylated antibody. Methods for PEGylating proteins are known in the art and can be applied to the antibodies of the present application. See, e.g., EPO 154 316 and EP 0 401 384.

The antibodies or the antigen-binding portions thereof of the present application can be characterized by their various physical properties, such that their classes are detected and/or distinguished.

For example, the antibodies or the antigen-binding portions may comprise one or more glycosylation sites in the light or heavy chain variable regions. These glycosylation sites may result in increased immunogenicity of the antibody or altered antibody pK values due to changed antigen binding. Glycosylation is known to occur in motifs containing N-X-S/T sequences. In some cases, it is preferred that the PD-L1 antibody or the antigen-binding portion thereof does not contain variable region glycosylation. This can be achieved by selecting an antibody that does not contain glycosylation motifs in the variable regions, or by mutating residues in the glycosylation region.

In preferred embodiments, the antibody or the antigen-binding portion does not contain an asparagine isomerization site. Deamidation of asparagine may occur in the N-G or D-G sequence, creating isoaspartate residues that introduce a kink into the polypeptide chain and reduce its stability (the isoaspartate effect).

Each antibody or antigen-binding portion thereof will have a unique isoelectric point (pI), substantially falling within a pH range of 6 to 9.5. The pI of IgG1 antibodies generally falls within a pH range of 7 to 9.5, whereas the pI of IgG4 antibodies substantially falls within a pH range of 6 to 8. It is speculated that antibodies with pI outside the normal range may have some unfolded structures and be unstable under *in vivo* conditions. Therefore, it is preferred that the pI value of the PD-L1 antibody falls within the normal range. This can be achieved by selecting antibodies with pI within the normal range or by mutating uncharged surface residues.

The monoclonal antibodies of the present application can be prepared using phage display technology. Phage display technology involves inserting a gene encoding an exogenous polypeptide or protein (e.g., an antibody in the form of scFv) into an appropriate position of the phage coat protein structural gene by genetic engineering techniques, such that the exogenous polypeptide or protein is correctly expressed in the reading frame. As a result, the exogenous polypeptide or protein (e.g., scFv) forms a fusion protein on the phage capsid protein and is displayed on the phage surface upon reassembly of progeny phages. Subsequently, by using a target molecule (e.g., PD-L1) and adopting an appropriate panning method, phages that do not specifically bind to the target molecule are washed away. The bound phages are then eluted using an acid, base, or competing molecule, and the neutralized phages are used to infect *Escherichia coli* for amplification. After 3 to 5 rounds of enrichment, the proportion of phages capable of specifically recognizing the target molecule is gradually increased, and finally polypeptides or proteins that recognize the target molecule are obtained.

Other methods for preparing the monoclonal antibodies include somatic cell hybridization (hybridoma), viral or oncogenic transformation of B lymphocytes, etc. Methods for preparing chimeric antibodies are also well known in the art. The antibodies or the antigen-binding portions thereof of the present application can also be produced in host cell transfectomas using, for example, recombinant DNA techniques in conjunction with gene transfection methods (see, e.g., Morrison, S. (1985) Science 229: 1202). In one embodiment, DNA encoding partial or full-length light and heavy chains obtained by standard molecular biology techniques is inserted into one or more expression vectors such that the genes are operably linked to transcriptional and translational regulatory sequences. In this case, the term "operably linked" refers to the linkage of the antibody genes into the vector such that the transcriptional and translational control sequences within the vector perform their intended function of regulating the transcription and translation of the antibody genes.

The term "regulatory sequence" includes promoters, enhancers, and other expression control elements (e.g., polyadenylation signals) that control the transcription or translation of the antibody genes. Preferred regulatory sequences for expression in mammalian host cells include viral elements that direct high-level protein expression in mammalian cells, e.g., promoters and/or enhancers derived from cytomegalovirus (CMV), simian virus 40 (SV40), adenovirus such as the adenovirus major late promoter (AdMLP), and polyomavirus. Alternatively, non-viral regulatory sequences can be used, such as the ubiquitin promoter or the β-globin promoter. In addition, the regulatory elements consist of sequences from different sources. For example, the SRα promoter system comprises sequences from the SV40 early promoter and the long terminal repeat of human T-cell leukemia virus type I. The expression vector and expression control sequences are selected to be compatible with the expression host cell used

The antibody light chain gene and the antibody heavy chain gene can be inserted into the same expression vector or different expression vectors. In preferred embodiments, variable regions are inserted into an expression vector encoding the heavy chain constant region and the light chain constant region of the desired subtype to construct a full-length antibody gene, such that the V_{H} is operably linked to the C_{H} in the vector and the V_{L} is operably linked to the C_{L} in the vector. Alternatively, the recombinant expression vector can encode a signal peptide that facilitates the secretion of antibody chains from the host cell. The antibody chain gene can be cloned into the vector such that the signal peptide is linked to the amino terminus of the antibody chain gene in the reading frame. The signal peptide may be an immunoglobulin signal peptide or a heterologous signal peptide (i.e., a signal peptide from a non-immunoglobulin protein).

In addition to the antibody chain genes and regulatory sequences, the recombinant expression vector of the present application may carry other sequences, e.g., a sequence that regulates the replication of the vector in the host cell (e.g., an origin of replication) and a selectable marker gene. The selectable marker gene can be used to select a host cell into which the vector has been introduced. For example, the selectable marker gene generally confers drug resistance to the host cell into which the vector has been introduced, e.g., resistance to G418, hygromycin, or methotrexate. Preferred selectable marker genes include the dihydrofolate reductase (DHFR) gene (for methotrexate selection/amplification in dhfr host cells) and the neo gene (for G418 selection).

For the expression of light and heavy chains, expression vectors encoding the heavy and light chains are transfected into host cells by standard techniques. The term "transfection" in its various forms encompasses a variety of techniques commonly used to introduce exogenous DNA into prokaryotic or eukaryotic host cells, e.g., electroporation, calcium phosphate precipitation, DEAE-dextran transfection, etc. Although the expression of the antibody or the antigen-binding portion thereof of the present application in prokaryotic or eukaryotic host cells is theoretically feasible, the antibody is preferably expressed in eukaryotic cells, and most preferably in mammalian host cells, because eukaryotic cells, particularly mammalian cells, are more likely than prokaryotic cells to assemble and secrete appropriately folded and immunologically active antibodies.

Preferred mammalian host cells for expressing the recombinant antibodies of the present application include Slc35C1 knockout cell lines, FUT8 knockout cell lines, the variant CHO cell line Lec13, the rat myeloma cell line YB2/0, cell lines comprising small interfering RNAs specifically directed against the FUT8 gene, cell lines co-expressing β-1,4-N-acetylglucosaminyltransferase III and Golgi α-mannosidase II, Chinese hamster ovary (CHO) cells (including dhfr-CHO cells used with the DHFR selectable marker), NSO myeloma cells, COS cells, and SP2 cells. When a recombinant expression vector encoding an antibody gene is introduced into a mammalian host cell, the antibody is prepared by culturing the host cell for a period of time sufficient to allow the expression of the antibody in the host cell, or preferably, sufficient to allow the secretion of the antibody into the culture medium in which the host cell is grown. The antibody or the antigen-binding portion thereof can be recovered from the culture medium using protein purification methods.

In another aspect, the present application provides a nucleic acid molecule encoding the heavy chain and/or light chain variable regions or CDRs of the antibody or the antigen-binding portion thereof of the present application. The nucleic acid may be present in an intact cell, in a cell lysate, or in a partially purified or substantially pure form. The nucleic acid is "isolated" or "substantially pure" when purified from other cellular components or other contaminants, such as other cellular nucleic acids or proteins, by standard techniques. The nucleic acid molecules of the present application may be, for example, DNA or RNA, and may or may not comprise intron sequences. In preferred embodiments, the nucleic acid is a cDNA molecule.

The nucleic acid of the present application can be obtained using standard molecular biology techniques. For antibodies expressed by hybridomas (e.g., hybridomas prepared from transgenic mice carrying human immunoglobulin genes), cDNAs encoding the light and heavy chains of the antibodies prepared from the hybridomas can be obtained by standard PCR amplification or cDNA cloning techniques. For antibodies obtained from an immunoglobulin gene library (e.g., using phage display technology), nucleic acids encoding such antibodies can be collected from the gene library.

Preferred nucleic acid molecules of the present application include those encoding the V_{H} and V_{L} sequences or CDRs of the PD-L1 monoclonal antibody. Once the DNA fragments encoding the V_{H} and V_{L} are obtained, operations such as conversion of the variable region genes to full-length antibody chain genes, Fab fragment genes, or scFv genes can be further performed on these DNA fragments by standard recombinant DNA techniques. In these operations, the DNA fragment encoding the V_{H} or V_{L} is operably linked to another DNA fragment encoding another protein, such as an antibody constant region or a flexible linker. The term "operably linked" means that two DNA fragments are linked together such that the amino acid sequences encoded by the two DNA fragments are both in the reading frame.

An isolated DNA encoding the V_{H} region can be converted into a full-length heavy chain gene by operably linking the V_{H}-encoding DNA to another DNA molecule encoding heavy chain constant region (C_{H1}, C_{H2}, and C_{H3}). The sequences of the human heavy chain constant region genes are known in the art, and DNA fragments comprising these regions can be obtained by standard PCR amplification. The heavy chain constant region may be an IgG1, IgG2, IgG3, IgG4, IgA, IgE, IgM, or IgD constant region, but is preferably an IgG1 constant region. For Fab fragment heavy chain genes, DNA encoding the V_{H} region can be operably linked to another DNA molecule encoding only the heavy chain C_{H1} constant region.

An isolated DNA encoding the V_{L} region can be converted into a full-length light chain gene by operably linking the V_{L}-encoding DNA to another DNA molecule encoding the light chain constant region C_{L}. The sequences of the human light chain constant region genes are known in the art, and DNA fragments comprising these regions can be obtained by standard PCR amplification. In preferred embodiments, the light chain constant region may be a κ or λ constant region.

To create an scFv gene, DNA fragments encoding V_{H} and V_{L} can be operably linked to another fragment encoding a flexible linker, such that the V_{H} and V_{L} sequences can be expressed as a contiguous single-chain protein, in which the V_{H} and V_{L} regions are linked via the flexible linker.

The antibody or the antigen-binding portion thereof of the present application can be conjugated to a therapeutic agent to form an immunoconjugate, e.g., an antibody-drug conjugate (ADC). Suitable therapeutic agents include cytotoxic molecules, alkylating agents, DNA minor groove binding molecules, DNA intercalators, DNA cross-linking agents, histone deacetylase inhibitors, nuclear export inhibitors, proteasome inhibitors, inhibitors of topoisomerase I or II, heat shock protein inhibitors, tyrosine kinase inhibitors, antibiotics, and antimitotic agents. In ADCs, the antibody and the therapeutic agent can be crosslinked via a linker that is cleavable, e.g., a peptide linker, a disulfide linker, or a hydrazone linker. More preferably, the linker is a peptide linker, e.g., Val-Cit, Ala-Val, Val-Ala-Val, Lys-Lys, Ala-Asn-Val, Val-Leu-Lys, Ala-Ala-Asn, Cit-Cit, Val-Lys, Lys, Cit, Ser, or Glu. ADCs can be prepared as described in U.S. Pat. Nos. 7,087,600; 6,989,452; and 7,129,261; PCT Publ. Nos. WO 02/096910; WO 07/038,658; WO 07/051,081; WO 07/059,404; WO 08/083,312; and WO 08/103,693; and U.S. Pat. Publ. Nos. 20060024317, 20060004081, and 20060247295. ADCs are applicable only in the context of antibody internalization. Internalizing antibodies can be conjugated to cytotoxic molecules such that the cytotoxic molecules specifically damage cells that internalize the antibody. In particular, cytotoxic molecules can enter target cells via antibody internalization. The cytotoxic molecules may be any small molecule compounds or protein molecules that damage target cells, e.g., tubulin polymerization inhibitors, DNA-damaging agents, etc.

In another aspect, the present application relates to a bispecific molecule comprising the antibody or the antigen-binding portion thereof of the present application linked to at least one other functional molecule, such as another peptide or protein (e.g., another antibody or a receptor ligand), to generate a bispecific molecule that binds to at least two different binding sites or target molecules. The term "bispecific molecule" includes molecules having three or more specificities.

Bispecific molecules may be present in a variety of forms and sizes. At one end of the size spectrum, bispecific molecules remain in the conventional antibody format, except that they have two binding arms with different types of specificity instead of having two binding arms with the same specificity. At the other extreme are bispecific molecules consisting of two single-chain antibody fragments (scFvs) linked via a peptide chain, referred to as Bs(scFv)₂ constructs. Bispecific molecules of intermediate size comprise two different F(ab) fragments linked via a peptide linker. These and other forms of bispecific molecules can be prepared by genetic engineering, somatic hybridization, or chemical methods.

The present application further provides a chimeric antigen receptor comprising a PD-L1 single-chain antibody scFv, wherein the scFv comprises the heavy chain and light chain CDRs, or the heavy chain and light chain variable regions described herein.

The PD-L1 chimeric antigen receptor may comprise: (a) an extracellular antigen-binding domain comprising a PD-L1 scFv; (b) a transmembrane domain; and (c) an intracellular signaling domain.

The present application further provides an immune cell, such as a T cell or NK cell, comprising the chimeric antigen receptor of the present application.

Oncolytic viruses preferentially infect and kill cancer cells. The antibody or the antigen-binding portion thereof of the present application can be used in combination with an oncolytic virus. Furthermore, an oncolytic virus encoding the antibody or the antigen-binding portion thereof of the present application can be introduced into a human body.

In another aspect, the present application provides a composition comprising the antibody or the antigen-binding portion thereof, the nucleic acid molecule, the expression vector, the host cell, the immunoconjugate, the chimeric antigen receptor, the immune cell, the bispecific antibody, and/or the oncolytic virus of the present application. In some embodiments, the composition is a pharmaceutical composition, which further comprises a pharmaceutically acceptable carrier. The composition may optionally comprise one or more other pharmaceutically active ingredients, e.g., another anti-tumor antibody or immune-enhancing antibody, or a non-antibody anti-tumor agent or immune-enhancing agent. The composition of the present application can be used in combination with, for example, another anti-cancer agent or another immune-enhancing agent.

The pharmaceutical composition may comprise any number of excipients. Excipients that can be used include carriers, surfactants, thickening or emulsifying agents, solid binders, dispersing or suspending agents, solubilizers, colorants, flavoring agents, coatings, disintegrants, lubricants, sweeteners, preservatives, isotonic agents, and combinations thereof. The selection and use of suitable excipients is taught in Gennaro, ed., Remington: The Science and Practice of Pharmacy, 20th Ed. (Lippincott Williams & Wilkins 2003).

Preferably, the pharmaceutical composition is suitable for intravenous, intramuscular, subcutaneous, parenteral, spinal, or epidermal administration (e.g., via injection or bolus injection). Depending on the route of administration, the active ingredient can be encapsulated in a material to protect it from acids and other natural conditions that may inactivate it. "Parenteral administration" refers to administration other than enteral and topical administration, and is generally performed via injection, including but not limited to intravenous, intramuscular, intraarterial, intramembranous, intracapsular, intraorbital, intracardiac, intradermal, intraperitoneal, transtracheal, subcutaneous, subcuticular, intraarticular, subcapsular, subarachnoid, intraspinal, epidural and intrasternal injection and bolus injection. Alternatively, the antibody of the present application can be administered via non-parenteral routes, e.g., external, epidermal, or mucosal administration, e.g., intranasal, oral, vaginal, rectal, sublingual, or topical administration.

The pharmaceutical compositions may be in the form of sterile aqueous solutions or dispersions. They may also be formulated in microemulsions, liposomes, or other ordered structures suitable for high concentrations of drugs.

The amount of active ingredient that is prepared together with a carrier material into a single dosage form will vary depending on a subject treated and a specific mode of administration, and is essentially the amount of the composition that produces a therapeutic effect. The amount of the active ingredient in combination with a pharmaceutically acceptable carrier is, by percentage, about 0.01% to about 99%.

The administration regimen is adjusted to provide the optimal desired response (e.g., a therapeutic response). For example, a bolus injection can be administered, multiple divided doses can be administered over time, or the dose can be reduced or increased in proportion to the severity of the condition being treated. It is particularly advantageous to formulate parenteral compositions in dosage unit forms for ease of administration and uniformity of dosage. Dosage unit form refers to physically discrete units suitable for single administration to a subject treated; each unit contains a predetermined amount of the active ingredient calculated to produce the desired therapeutic effect in association with the pharmaceutical carrier. Alternatively, the antibody can be administered as a sustained-release formulation, in which case the required frequency of administration is reduced.

For the administration of the antibody, the dosage may be about 0.001 to 100 mg/kg of host body weight. An exemplary therapeutic regimen involves administration once weekly.

A "therapeutically effective amount" of the pharmaceutical composition of the present application results in a reduction in the severity of disease symptoms and an increase in the frequency and duration of asymptomatic periods. For example, for the treatment of a subject with a tumor, a "therapeutically effective amount" inhibits tumor growth by at least about 20%, at least about 40%, even at least about 60%, and more particularly at least about 80%, as compared to an untreated subject. A therapeutically effective amount of a therapeutic antibody may reduce tumor size, or alleviate a symptom in a subject, which may be a human or another mammal.

The pharmaceutical composition may be a sustained-release agent, including implants and microencapsulated delivery systems. Biodegradable and biocompatible polymers such as ethylene-vinyl acetate, polyanhydrides, polyglycolic acid, collagen, polyorthoesters, and polylactic acid can be used. See, e.g., Sustained and Controlled Release Drug Delivery Systems, J. R. Robinson, ed., Marcel Dekker, Inc., New York, 1978.

The pharmaceutical compositions can be administered by a medical device, such as (1) a needleless hypodermic injection device (e.g., U.S. Pat. Nos. 5,399,163; 5,383,851; 5,312,335; 5,064,413; 4,941,880; 4,790,824; and 4,596,556); (2) a micro-infusion pump (U.S. Pat. No. 4,487,603); (3) a transdermal administration device (U.S. Pat. No. 4,486,194); (4) a bolus injection device (U.S. Pat. Nos. 4,447,233 and 4,447,224); and (5) an osmotic device (U.S. Pat. Nos. 4,439,196 and 4,475,196).

In certain embodiments, the components of the composition of the present application can be formulated to ensure appropriate *in vivo* distribution. For example, to ensure that the therapeutic antibody or the antigen-binding portion thereof of the present application crosses the blood-brain barrier, the antibody can be formulated in liposomes, which may additionally comprise targeting functional moieties to enhance selective delivery to specific cells or organs.

The present application further relates to *in vivo* gene therapy, wherein a nucleic acid molecule encoding the antibody or the antigen-binding portion thereof, the immunoconjugate, the bispecific molecule, etc., of the present application is directly introduced into a subject. For example, a nucleic acid sequence encoding the antibody or the antigen-binding portion thereof of the present application is introduced into a target cell by local injection via a nucleic acid construct with or without a suitable delivery vector, e.g., an adeno-associated virus vector. Other alternative viral vectors include, but are not limited to, retrovirus, adenovirus, herpes simplex virus, and papillomavirus vectors. *In vivo* physical transfer of viral vectors can be accomplished by local injection of a desired nucleic acid construct or other suitable delivery vector comprising the desired nucleic acid sequence, liposome-mediated transfer, direct injection (naked DNA), or particle bombardment (gene gun).

The pharmaceutical composition of the present application has a variety of *in vitro* and *in vivo* applications, involving, for example, the treatment of cancer, or more generally, immune enhancement in patients with diseases such as cancer. The pharmaceutical composition can be administered to a human subject to, for example, inhibit tumor growth *in vivo*.

In view of the ability of the pharmaceutical composition of the present application to inhibit the proliferation and survival of tumor cells, the present application provides a method for inhibiting the growth of tumor cells in a subject, which comprises administering to the subject the pharmaceutical composition of the present application, thereby inhibiting tumor growth in the subject. Nonlimiting examples of tumors that can be treated with the antibody of the present application include, but are not limited to, solid tumors. The solid tumors include, but are not limited to, melanoma, non-small cell lung cancer, renal cell carcinoma, bladder cancer, or head and neck squamous cell carcinoma, whether primary or metastatic. Furthermore, refractory or recurrent malignant tumors may also be treated with the pharmaceutical compositions of the present application.

The present application provides a combination therapy comprising administering the pharmaceutical composition of the present application with one or more other antibodies or non-antibody therapeutic agents, which is effective in inhibiting tumor growth in a subject. In one embodiment, the present application provides a method for inhibiting tumor growth in a subject, which comprises administering to the subject the pharmaceutical composition of the present application and one or more other antibodies, e.g., a PD-1 antibody. In certain embodiments, the subject is a human. In another aspect, the present application provides a method for treating cancer, wherein the pharmaceutical composition of the present application is administered with a chemotherapeutic agent, which may be a cytotoxic agent. Other therapies that can be combined with the pharmaceutical composition of the present application include, but are not limited to, administration of an immunogenic agent, administration of interleukin-2 (IL-2), radiotherapy, surgery, or hormone ablation.

The composition of the present application may also be used to reverse or alleviate immunosuppression in a subject. In particular, the present application further provides a method for reversing or alleviating immunosuppression in a subject, the method comprising administering to the subject an effective amount of the pharmaceutical composition of the present application. In some embodiments, the method is used for reversing or alleviating immunosuppression in the tumor microenvironment, the method comprising administering to a tumor site an effective amount of the pharmaceutical composition of the present application.

The composition of the present application may also be used to enhance an immune response. Specifically, the present application provides a method for enhancing an immune response in a subject, the method comprising administering to the subject an effective amount of the pharmaceutical composition of the present application.

The combination of therapeutic agents discussed herein can be administered simultaneously as a single composition in a pharmaceutically acceptable carrier, or administered simultaneously as separate compositions, wherein each agent is in a pharmaceutically acceptable carrier. In another embodiment, the combination of therapeutic agents can be administered sequentially.

Furthermore, if the combination therapy is administered multiple times and the agents are administered sequentially, the sequence in the sequential administration at each time point can be reversed or maintained, and the sequential administration can be combined with simultaneous administration or any combination thereof.

Various aspects and embodiments of the present application will be discussed with reference to the accompanying drawings and the following examples. Other aspects and embodiments will be apparent to those skilled in the art. All documents described herein are incorporated herein by reference in their entireties. Although the present application has been described in conjunction with exemplary embodiments, many equivalent modifications and variations will be apparent to those skilled in the art from the present application. Accordingly, the exemplary embodiments of the present application are intended to be illustrative and not restrictive. Various modifications can be made to the embodiments without departing from the spirit and scope of the present application.

### Example 1. Acquisition of positive phage clones expressing anti-human PD-L1 antibody

A natural fully human phage display library was subjected to three rounds of panning against biotinylated human PD-L1 protein (ACROBiosystems, PD1-H82E5), a cell line stably expressing human PD-L1 (Nanjing Probio, RD00868), and a CHO-K1 cell line (Nanjing Probio), to obtain a phage library panning eluate. The neutralized phage panning eluate was added to the prepared TG1 bacterial solution, mixed uniformly, and then left to stand at 37°C for 45 minutes to infect TG1 host bacteria. After sufficient infection, the bacterial solution was serially diluted, spread onto an agar plate containing the relevant antibiotic, and then cultured overnight at 37°C in an inverted state. The next day, a sterile 96-well deep-well plate containing 0.5 mL of 2YT medium (supplemented with 0.2% w/v glucose and 0.1 mg/mL ampicillin) per well was prepared. Monoclonal colonies grown on the plate were picked using a sterile tip into corresponding wells, and then cultured overnight at 37°C with shaking at 220 rpm for 16 to 18 hours. The next day, 0.05 to 0.1 mL of the overnight cultured monoclonal bacterial solution was transferred into a newly prepared sterile deep-well plate containing 0.40 to 0.45 mL of 2YT medium (supplemented with ampicillin at a final concentration of 0.1 mg/mL) per well. The bacterial solution was cultured until the OD₆₀₀ value reached about 0.6 to 0.8, and then helper phage was added at an appropriate ratio. After uniform mixing with shaking, the mixture was left to stand at 37°C for 45 minutes for infection. Subsequently, 0.25 mL of 2YT medium (containing 0.1 mg/mL ampicillin, and kanamycin at a final concentration of 0.05 mg/mL after addition) was supplemented, and then the mixture was cultured overnight at 30°C with shaking at 220 rpm for 16 to 18 hours. The overnight-expressed bacterial solution was centrifuged at 4000 rpm for 10 minutes to obtain a phage display expression supernatant, which was used for indirect ELISA binding assay against human PD-L1 protein (GenScript, Z03425) and FACS binding assay against PD-L1-expressing cells, so as to obtain positive phage clones expressing fully human antibodies.

Specifically, in the indirect ELISA, 100 µl/well of 1 µg/mL recombinant human PD-L1-His protein (GenScript, Z03425) in carbonate buffer solution (CBS) as a coating reagent was added to an ELISA microplate, followed by coating overnight at 4°C. The plate was washed with PBST (containing 0.05% Tween) and blocked with 300 µl/well of PBS containing 3% skimmed milk at 37°C for 1 hour. Subsequently, the blocking solution was discarded. 50 µl of phage expression supernatant and 50 µl of 0.05% PBST were added to the plate, and then the plate was incubated at room temperature for 2 hours. The plate was washed three times with 0.05% PBST, and incubated with 100 µl/well of horseradish peroxidase-conjugated goat anti-M13 phage antibody (Sino Biological) at room temperature for 45 minutes. The plate was washed six times with 0.05% PBST. Then, TMB substrate solution (GenScript) was added, and the plate was incubated at room temperature in the dark for 10 minutes. Then, 50 µl of 1 M HCl stop solution (Sigma) was added to terminate the reaction. The plate was read at 450 nm using a microplate reader.

In FACS, the above PD-L1⁺ cells stably expressing human PD-L1 (Probio, RD00868) and PD-L1⁻ CHO-K1 cells were collected and washed three times with PBS. 1.0 × 10⁵ PD-L1⁺ cells or CHO-K1 cells, 50 µl of the test supernatant, and 50 µl of 3.5 µg/mL biotinylated anti-phage antibody were added sequentially to a 96-well plate, and then the plate was incubated at 4°C for 1 hour. Subsequently, the cells were washed three times with PBS. 100 µl of iFluor-labeled streptavidin (Jackson, 016-600-084) was added, and then the plate was incubated at 4°C for 45 minutes. Finally, the cells were washed three times with PBS, and signals were read using FACS Calibur (BD).

### Example 2. Sequencing of variable regions of monoclonal PD-L1 antibodies and recombinant production of antibodies

Based on the results of ELISA and FACS assays in Example 1, the bacterial solutions corresponding to positive clones were subjected to culture, phagemid extraction, PCR, and monoclonal Sanger sequencing. Finally, three positive clones were obtained. The antibody IDs and antibody sequences/sequence numbers corresponding to the three clones are shown in Table 1.

**Table 1. Positive clones and sequence numbers of their corresponding antibodies**

| Antibody ID | | CDR1 region Amino acid sequence | CDR2 region Amino acid sequence | CDR3 region Amino acid sequence | Variable region Amino acid sequence |
|---|---|---|---|---|---|
| AHP300 05 | Heavy chain | SEQ ID NO: 1 | SEQ ID NO: 2 | SEQ ID NO: 3 | SEQ ID NO: 6 |
| | Light chain | SEQ ID NO: 4 | DAS | SEQ ID NO: 5 | SEQ ID NO: 7 |
| AHP313 19 | Heavy chain | SEQ ID NO: 8 | SEQ ID NO: 9 | SEQ ID NO: 10 | SEQ ID NO: 13 |
| | Light chain | SEQ ID NO: 11 | GAS | SEQ ID NO: 12 | SEQ ID NO: 14 |
| AHP300 19 | Heavy chain | SEQ ID NO: 8 | SEQ ID NO: 9 | SEQ ID NO: 10 | SEQ ID NO: 17 |
| | Light chain | SEQ ID NO: 15 | EVS | SEQ ID NO: 16 | SEQ ID NO: 18 |

Codon-optimized variable region DNA fragments were synthesized and inserted into the pcDNA3.4-Fc(HuIgG1) expression vector to construct an expression plasmid.

The above plasmid was transfected into ExpiCHO-S cells, and after culturing in a shake flask at 37°C for 7 days, the supernatant was collected for antibody purification. Before purification, tubing and Protein A column were depyrogenated with 0.2 M NaOH, and the column was re-equilibrated with a buffer containing 0.05 M Tris and 1.5 M NaCl (pH = 8.0). Subsequently, the harvested cell culture supernatant was diluted at a 1 : 1 ratio with 2× the above buffer and sterilized by filtration. The filtered supernatant was incubated with the Protein A column at room temperature for 2 hours. After washing the column with 1× the above buffer, the antibody was eluted using sterile 0.1 M sodium citrate (pH 3.5). The collected eluate was neutralized with one-ninth the volume of sterile 1 M Tris-HCl (pH = 9.0). Under sterile conditions, the product buffer was exchanged to PBS (pH 7.4) by dialysis to remove any residual elution buffer, and the resulting sample was concentrated. After concentration, antibody quantification was performed at OD280 nm using an extinction coefficient Ec (0.1%) of 1.43.

The purified antibody was analyzed by SDS-PAGE using a 10% precast gel (GenScript) on a BioRad electrophoresis system. The gel was stained with Estain 2.0 (GenScript), and the molecular weight and purity were estimated by comparing the stained bands.

The heavy chain of the expressed antibody comprises a heavy chain variable region and a heavy chain constant region (Uniprot Accession No. P01857-1), and the light chain comprises a light chain variable region and a light chain constant region (Uniprot Accession No. P01834).

### Example 3. Binding affinity of monoclonal antibodies for recombinant human PD-L1 protein

Indirect ELISA was used to evaluate the binding ability of the purified antibodies of the present application to recombinant human PD-L1-His protein.

Specifically, an ELISA plate was coated with 100 µl/well of 1 µg/mL recombinant human PD-L1 protein (GenScript, Z03425) in carbonate buffer solution (CBS, prepared in-house by Probio) overnight at 4°C. The ELISA plate was washed with PBST (containing 0.05% Tween) and blocked with PBS containing 3% skimmed milk at 37°C for 1 hour. Subsequently, the blocking solution was discarded. 100 µl of 3-fold serially diluted purified antibody of the present application, Avelumab (synthesized by Nanjing Probio), or IgG1 isotype control antibody, with an initial concentration of 15 µg/mL (about 100 nM) across a total of 11 test concentration gradients, was added to the plate. The plate was incubated at 37°C for 1 hour, washed three times with 0.05% PBST, and incubated with 100 µl/well of horseradish peroxidase-conjugated mouse anti-human IgG Fc fragment (GenScript, A01854) at 37°C for 0.5 hour. The plate was washed six times with 0.05% PBST. Then, TMB substrate solution (GenScript) was added, and the plate was incubated at room temperature in the dark for 15 minutes. Subsequently, 50 µl of 1 M HCl stop solution (Sigma) was added to terminate the reaction. The plate was read at 450 nm using a microplate reader.

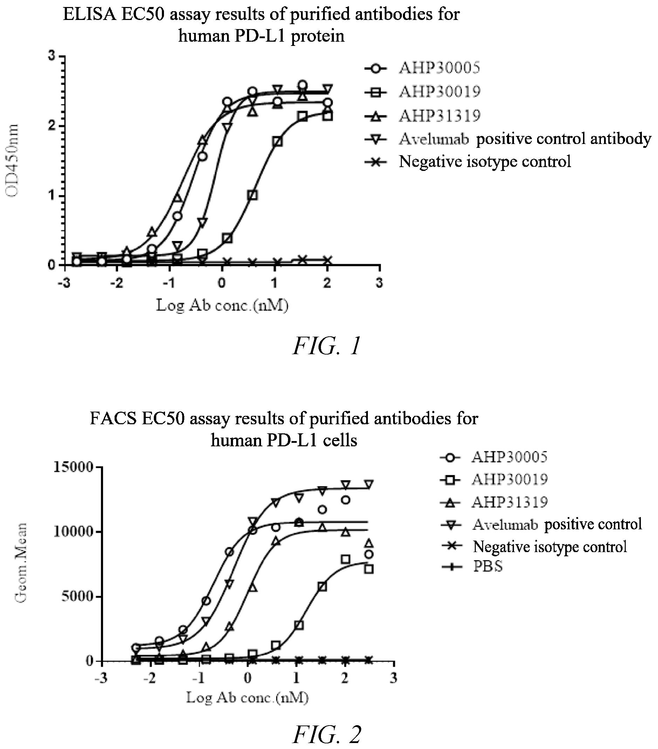

The ELISA results for the binding of the three antibodies of the present application to PD-L1 protein are presented in FIG. 1, and the EC₅₀ values of the antibodies are shown in Table 2. It can be seen that, as compared to Avelumab, the PD-L1 antibodies of the present application exhibit comparable binding affinity for recombinant human PD-L1 antigen protein.

**Table 2. Binding affinity assay of PD-L1 antibodies to PD-L1 protein**

| Antibody | ELISA EC₅₀ (nM) |
|---|---|
| AHP30005 | 0.2752 |
| AHP30019 | 4.261 |
| AHP31319 | 0.1731 |
| Avelumab | 0.7349 |

### Example 4. Binding affinity of monoclonal antibodies for cells expressing human, cynomolgus monkey, and mouse PD-L1

Stable cell lines expressing human, cynomolgus monkey, and mouse PD-L1 protein (Probio, RD00868; Probio, M00573; Probio, M00567), and PD-L1⁻ CHO-K1 cells were collected separately and washed three times with PBS. 1 × 10⁵ test cells and three-fold serially diluted purified antibody or control antibody with an initial concentration of 45 µg/mL (about 300 nM) were added to a 96-well plate, in a total volume of 100 µl per well, and then the plate was incubated at 4°C for 1 hour. Subsequently, the cells were washed three times with PBS. 100 µl of iFluor-labeled goat anti-human IgG Fcγ-specific antibody (Jackson, 115-545-071) was added, and then the plate was incubated at 4°C for 45 minutes. Finally, the cells were washed three times with PBS, and signals were read using a FACS analyzer (BD Calibur).

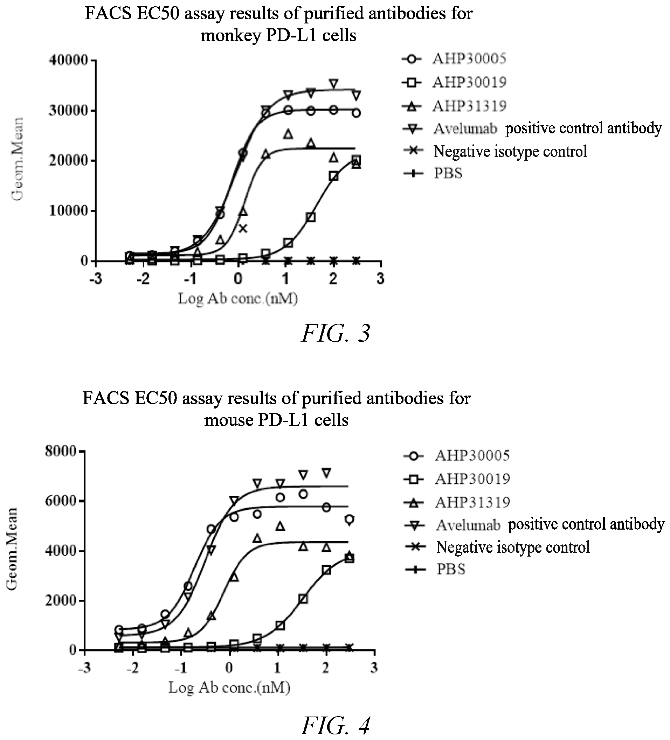

FIGs. 2-4 show the binding assay results of the antibodies to stable cell lines expressing human, cynomolgus monkey, and mouse PD-L1 protein, respectively. The EC₅₀ values are shown in Table 3. It can be seen that all antibodies of the present application bind to cells expressing human, cynomolgus monkey, and mouse PD-L1, but not to PD-L1-negative cells, indicating that the binding of the three antibodies of the present application to human, cynomolgus monkey, and mouse PD-L1 cells is specific.

**Table 3. FACS EC₅₀ Assay of Fully Human Antibodies to PD-L1-Associated Cells**

| Antibody | Human PD-L1 protein FACS EC₅₀ (nM) | Monkey PD-L1 protein FACS EC₅₀ (nM) | Mouse PD-L1 protein FACS EC₅₀ (nM) |
|---|---|---|---|
| AHP30005 | 0.193 | 0.7248 | 0.1841 |
| AHP30019 | 15.89 | 43.46 | 31.79 |
| AHP31319 | 0.9765 | 1.343 | 0.7458 |
| Avelumab | 0.5114 | 0.8943 | 0.3062 |

### Example 5. SPR binding assay of monoclonal antibodies to human PD-L1 protein

The binding affinity of the antibodies of the present application to recombinant human PD-L1 protein was determined using a surface plasmon resonance (SPR) biosensor, Biacore 8K (Cytiva).

Specifically, the antibodies were immobilized on the sensor chip using the Fc capture method, and human PD-L1 protein (GenScript, Z03425) was used as the analyte. Seven different concentrations of human PD-L1 (concentration range: 0.78125 to 200 nM) were injected into the flow cells at a flow rate of 30 µl/min for 120 seconds to measure binding. Subsequently, buffer was injected to allow dissociation for 360 seconds to measure the dissociation of the antibody from the antigen protein. The dissociation (kd) and association (ka) rate constants were obtained using the Biacore 8K evaluation software, and the equilibrium dissociation constant (K_{D}) was calculated as the ratio of kd to ka.

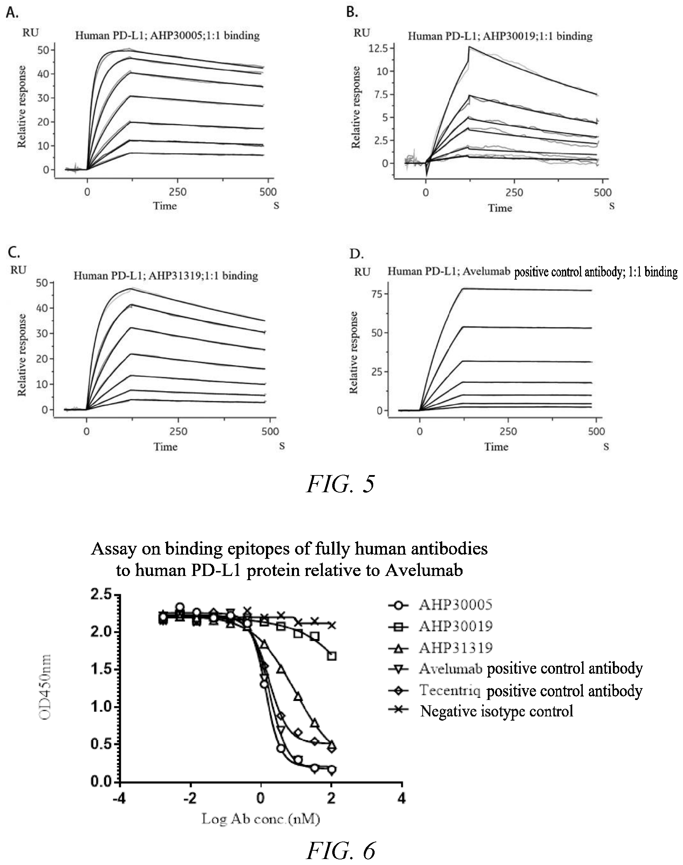

The SPR affinity determination results are shown in Table 4, and the corresponding sensorgrams are presented in FIG. 5 (A-D). It can be seen that the antibodies of the present application and Avelumab exhibit comparable binding affinity levels to the human PD-L1 antigen protein. In particular, AHP30005 shows an equilibrium dissociation constant (K_{D}) in the 10⁻¹⁰ range.

**Table 4. Affinity determination of fully human antibodies to human PD-L1 protein**

| Antibody | Ka (1/Ms) | Kd (1/s) | K_{D} (M) | Rmax (RU) | Chi² (RU²) |
|---|---|---|---|---|---|
| AHP30005 | 1.39E+06 | 4.25E-04 | 3.05E-10 | 48.7 | 2.65E-01 |
| AHP30019 | 2.90E+04 | 1.46E-03 | 5.03E-08 | 17.8 | 6.81E-02 |
| AHP31319 | 1.67E+05 | 8.48E-04 | 5.08E-09 | 47.2 | 1.28E-01 |
| Avelumab | 4.49E+04 | 3.74E-05 | 8.34E-10 | 79.2 | 6.16E-02 |

### Example 6. Binding epitopes of monoclonal antibodies on human PD-L1 protein

Indirect ELISA was used to determine whether the binding epitopes of the antibodies in the present application on human PD-L1-His protein (GenScript, Z03425) are identical to those of Avelumab and Tecentriq (synthesized by Nanjing Probio).

Specifically, an ELISA plate was coated with 100 µl/well of 1 µg/mL recombinant human PD-L1(His) protein (GenScript, Z03425) in CBS (prepared in-house by Probio) overnight at 4°C. The plate was washed with PBST (containing 0.05% Tween) and blocked with 300 µl/well of PBS containing 3% skimmed milk at 37°C for 1 hour. Subsequently, the blocking solution was discarded. 50 µl of 3-fold serially diluted antibodies of the present application or control antibodies, with an initial concentration of 15 µg/mL (about 100 nM) across a total of 11 test concentration gradients, were added to the plate, and then the plate was incubated at 37°C for 1 hour. Then, 50 µl of 0.02 µg/mL biotinylated Avelumab or Tecentriq was added to the plate, and then the plate was incubated at 37°C for 1 hour. The plate was washed three times with PBST, and incubated with 100 µl/well of horseradish peroxidase-conjugated avidin antibody (GenScript, M00091) at 37°C for 0.5 hour. The plate was washed six times with PBST. Then, TMB substrate solution (GenScript) was added, and the plate was incubated at room temperature in the dark for 10 minutes. Then, 50 µl of 1 M HCl stop solution (Sigma) was added to terminate the reaction. The plate was read at 450 nm using a microplate reader.

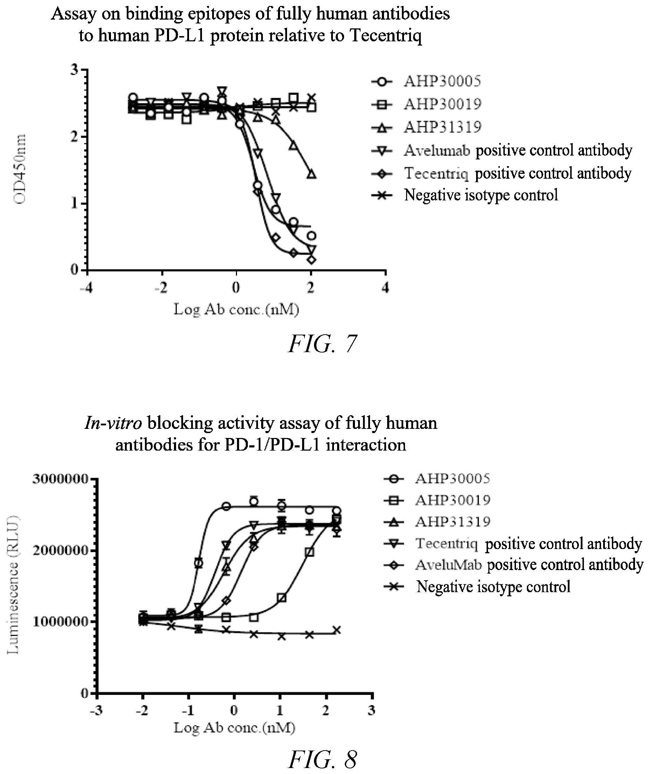

FIG. 6 shows the detection results after addition of biotinylated Avelumab, and FIG. 7 shows the detection results after addition of biotinylated Tecentriq. The IC₅₀ values for inhibition of the binding of the antibodies to human PD-L1 by Avelumab and Tecentriq are shown in Table 5.

**Table 5. Assay on binding epitopes of fully human antibodies to human PD-L1 protein**

| Antibody | Avelumab ELISA IC₅₀ (nM) | Tecentriq ELISA IC₅₀ (nM) |
|---|---|---|
| AHP30005 | 1.344 | 2.807 |
| AHP30019 | ~ 2.219e+009 | 0.972 |
| AHP31319 | 8.974 | 96.12 |
| Avelumab | 1.736 | 6.607 |
| Tecentriq | 1.675 | 3.329 |

It can be seen from the results that Avelumab can effectively inhibit the binding of AHP30005 and AHP31319 to human PD-L1 protein, while having little effect on the binding of AHP30019 to human PD-L1 protein. This indicates that the binding epitopes of AHP30005 and AHP31319 on PD-L1 likely overlap with those of Avelumab, whereas the binding epitope of AHP30019 likely does not overlap or barely overlaps with that of Avelumab.

Furthermore, Tecentriq can effectively inhibit the binding of AHP30005 to human PD-L1 protein, and also has an effect on the binding of AHP31319 to human PD-L1 protein, while having almost no effect on the binding of AHP30019 to human PD-L1 protein. This indicates that the binding epitope of AHP30005 on PD-L1 likely overlaps with that of Tecentriq, and the binding epitope of AHP31319 on PD-L1 also likely overlaps with that of Tecentriq, whereas the binding epitope of AHP30019 should not overlap with that of Tecentriq.

### Example 7. In-vitro blocking activity of monoclonal antibodies against PD-1 and PD-L1

Using Jurkat cells expressing human PD-1 protein and an NFAT-RE-induced luciferase reporter gene (GS-J2B/PD-1 cell line, Probio, RD00871), and CHO-K1 cells expressing human PD-L1 and cell surface proteins capable of activating TCR in a non-antigen-dependent manner (GS-C3/PD-L1 cell line, Probio, RD00703), the blocking effect of the antibodies of the present application on the PD-1/PD-L1 interaction was determined using a PD-1/PD-L1 blockade bioassay. When the above Jurkat cells are co-cultured with GS-C3/PD-L1 cells, PD-L1 on the GS-C3/PD-L1 cells interacts with PD-1 on the Jurkat cells, thereby inhibiting TCR signaling and NFAT-RE-driven luciferase activity in Jurkat cells. In contrast, after addition of a PD-L1 antibody that blocks the PD-1/PD-L1 interaction, the inhibitory signal is relieved, thereby triggering activation of the TCR signaling pathway and luminescence of the NFAT-RE-driven luciferase.

Specifically, the culture medium was removed from GS-C3/PD-L1 cells, and the cells were washed with 2 mL of DPBS. 2 mL of trypsin was added, and the cells were digested at 37°C for 5 minutes. Then, 8 mL of cell culture medium was added to terminate the digestion, and the cell suspension was collected and centrifuged at 800 rpm for 5 minutes. The cells were gently resuspended in 1 mL of F12K cell culture medium (Thermo Fisher, 21127022) to prepare a single-cell suspension, which was then diluted to about 1.25 × 10⁵ cells/mL. 40 µl of the cell suspension was transferred to a 384-well white flat-bottom assay plate, which was then incubated overnight (16 to 20 hours) in an incubator at 37°C with 5% CO₂. The culture medium was removed from Jurkat cells, and the cells were washed with 2 mL of DPBS. 2 mL of trypsin was added, and the cells were digested at 37°C for 5 minutes. Then, 8 mL of cell culture medium was added to terminate the digestion, and the cell suspension was collected and centrifuged at 800 rpm for 5 minutes. The cells were gently resuspended in 1000 µl of complete medium to prepare a single-cell suspension, which was then diluted to about 7.5 × 10⁵ cells/mL. The 384-well assay plate containing GS-C3/PD-L1 cells was removed from the incubator. The assay plate was inverted over a sink to remove the culture medium, and then placed upside down on a paper towel for 5 to 10 seconds to absorb the remaining culture medium. 20 µl of three-fold serially diluted antibodies of the present application or control antibodies with an initial concentration of 170 nM and 20 µl of Jurkat cell suspension were added to the 384-well assay plate containing GS-C3/PD-L1 cells, and then the plate was incubated at 37°C with 5% CO₂ for 6 hours. 40 µl of Bio-Lite luciferase assay system reagent was added, and the signal was read using a Pherastar microplate reader.

The results are shown in FIG. 8. It can be seen that all antibodies of the present application can inhibit the PD-1/PD-L1 interaction in a concentration-dependent manner. Among them, the antibody AHP30005 exhibits the optimal inhibitory effect, which is superior to that of Tecentriq and Avelumab, and the inhibitory effect of AHP31319 is superior to that of Avelumab.

The amino acid and nucleotide sequence information mentioned herein is as follows.
(1) AHP30005 fully human antibody
   Heavy chain variable region VH CDR1
      GRTALTYA (SEQ ID NO: 1)
   Heavy chain variable region VH CDR2
      INWSGSMT (SEQ ID NO: 2)
   Heavy chain variable region VH CDR3
      AATRTAITMPPQRDGVDY (SEQ ID NO: 3)
   Heavy chain variable region 
QVQLVDSGGGLVQAGGSLSVSCAVSGRTALTYAVGWFRQAPGKERDFVA
GINWSGSMTNYADSVKGRFTISRDNQKNTVYLQMNSLKPEDTAVYYCAA
TRTAITMPPQRDGVDYWGKGTTVTVSS (SEQ ID NO: 6)
   Light chain variable region VL CDR1
      QDISNY (SEQ ID NO: 4)
   Light chain variable region VL CDR2
      DAS
   Light chain variable region VL CDR3
      QQYDNLPRT (SEQ ID NO: 5)
   Light chain variable region 
VIWMTQSPSSLSASVGDRVTITCQASQDISNYLNWYQQKPGKAPKLLIYDA
SNLETGVPSRFSGSGSGTDFTFTISSLQPEDIATYYCQQYDNLPRTFGQGTK
VEIK (SEQ ID NO: 7)
(2) AHP31319 fully human antibody
   Heavy chain variable region VH CDR1
      GSSFSLAV (SEQ ID NO: 8)
   Heavy chain variable region VH CDR2
      ISSAAGT (SEQ ID NO: 9)
   Heavy chain variable region VH CDR3
      WRGAVPGDPYGR (SEQ ID NO: 10)
   Heavy chain variable region 
EVQLVQSGGGLVQPGGSLRLSCAGSGSSFSLAVMGWYRQAPGRQRELVGI
ISSAAGTNYADSVKGRFTISRDDAKNTLYLQMNSLTAEDTAVYYCWRGA
VPGDPYGRWGQGTTVTVSS (SEQ ID NO: 13)
   Light chain variable region VL CDR1
      QSVSNNY (SEQ ID NO: 11)
   Light chain variable region VL CDR2
      GAS
   Light chain variable region VL CDR3
      QQYGNSPGT (SEQ ID NO: 12)
   Light chain variable region 
EIVMTQSPGTLSLSPGEGATLSCRASQSVSNNYLAWYQQKHGQAPRLLIYG
ASSRATGIPDRFSGSGSGTDFTLTINRLEPEDFALYYCQQYGNSPGTFGQGT
KVDIK (SEQ ID NO: 14)
(3) AHP30019 fully human antibody
   Heavy chain variable region VH CDR1
      GSSFSLAV (SEQ ID NO: 8)
   Heavy chain variable region VH CDR2
      ISSAAGT (SEQ ID NO: 9)
   Heavy chain variable region VH CDR3
      WRGAVPGDPYGR (SEQ ID NO: 10)
   Heavy chain variable region 
QVQLVQSGGGLVQPGGSLRLSCAGSGSSFSLAVMGWYRQAPGRQRELVGI
ISSAAGTNYADSVKGRFTISRDDAKNTLYLQMNSLTAEDTAVYYCWRGA
VPGDPYGRWGQGTLVTVSS (SEQ ID NO: 17)
   Light chain variable region VL CDR1
      QSLVHSDGNTY (SEQ ID NO: 15)
   Light chain variable region VL CDR2
      EVS
   Light chain variable region VL CDR3
      MQGTHWPWT (SEQ ID NO: 16)
   Light chain variable region

EIVLTQSPLSLPVTLGQPASISCRSSQSLVHSDGNTYLNWFQQRPGQPPRRLI
YEVSNRDSGVPDRFSGSGSGTDFTLKISRVEAEDVGVYYCMQGTHWPWTF
GQGTKVEIK (SEQ ID NO: 18)

Although the present application has been described in conjunction with one or more embodiments, it should be understood that the present application is not limited to these embodiments. The description in the present application is intended to cover all variations and equivalents, all of which are included within the spirit and scope of the appended claims. All documents cited herein are incorporated by reference in their entireties.

## Claims

1. An isolated monoclonal antibody or an antigen-binding portion thereof that is capable of specifically binding to PD-L1, **characterised in that** the antibody or the antigen-binding portion thereof comprises:
i) a heavy chain variable region comprising VH CDR1, VH CDR2, and VH CDR3, wherein VH CDR1, VH CDR2, and VH CDR3 comprise amino acid sequences of: (1) GRTALTYA (SEQ ID NO: 1), INWSGSMT (SEQ ID NO: 2), and AATRTAITMPPQRDGVDY (SEQ ID NO: 3), respectively; or (2) GSSFSLAV (SEQ ID NO: 8), ISSAAGT (SEQ ID NO: 9), and WRGAVPGDPYGR (SEQ ID NO: 10), respectively; or amino acid sequences having 1 to 3 amino acid substitutions in each CDR as compared to the above amino acid sequences; and/or
ii) a light chain variable region comprising VL CDR1, VL CDR2, and VL CDR3, wherein VL CDR1, VL CDR2, and VL CDR3 comprise amino acid sequences of: (1) QDISNY (SEQ ID NO: 4), DAS, and QQYDNLPRT (SEQ ID NO: 5), respectively; (2) QSVSNNY (SEQ ID NO: 11), GAS, and QQYGNSPGT (SEQ ID NO: 12), respectively; or (3) QSLVHSDGNTY (SEQ ID NO: 15), EVS, and MQGTHWPWT (SEQ ID NO: 16), respectively; or amino acid sequences having 1 to 3 amino acid substitutions in each CDR as compared to the above amino acid sequences.

2. The isolated monoclonal antibody or the antigen-binding portion thereof according to claim 1, **characterised in that** the antibody or the antigen-binding portion thereof comprises a heavy chain variable region and a light chain variable region, wherein VH CDR1, VH CDR2, VH CDR3, VL CDR1, VL CDR2 and VL CDR3 comprise amino acid sequences of: (1) GRTALTYA (SEQ ID NO: 1), INWSGSMT (SEQ ID NO: 2), AATRTAITMPPQRDGVDY (SEQ ID NO: 3), QDISNY (SEQ ID NO: 4), DAS, and QQYDNLPRT (SEQ ID NO: 5), respectively; (2) GSSFSLAV (SEQ ID NO: 8), ISSAAGT (SEQ ID NO: 9), WRGAVPGDPYGR (SEQ ID NO: 10), QSVSNNY (SEQ ID NO: 11), GAS, and QQYGNSPGT (SEQ ID NO: 12), respectively; or (3) GSSFSLAV (SEQ ID NO: 8), ISSAAGT (SEQ ID NO: 9), WRGAVPGDPYGR (SEQ ID NO: 10), QSLVHSDGNTY (SEQ ID NO: 15), EVS, and MQGTHWPWT (SEQ ID NO: 16), respectively.

3. The isolated monoclonal antibody or the antigen-binding portion thereof according to claim 1 or 2, **characterised in that** the heavy chain variable region comprises an amino acid sequence having at least 80%, 85%, 90%, 91%, 92%, 93%, 94%, 95%, 96%, 97%, 98%, 99%, or 100% sequence identity to SEQ ID NO: 6, 13, or 17.

4. The isolated monoclonal antibody or the antigen-binding portion thereof according to any one of claims 1 to 3, **characterised in that** the light chain variable region comprises an amino acid sequence having at least 80%, 85%, 90%, 91%, 92%, 93%, 94%, 95%, 96%, 97%, 98%, 99%, or 100% sequence identity to SEQ ID NO: 7, 14, or 18.

5. The isolated monoclonal antibody or the antigen-binding portion thereof according to any one of claims 2 to 4, **characterised in that** the heavy chain variable region and the light chain variable region comprise amino acid sequences having at least 80%, 85%, 90%, 91%, 92%, 93%, 94%, 95%, 96%, 97%, 98%, 99%, or 100% sequence identity to: (1) SEQ ID NOs: 6 and 7, respectively; (2) SEQ ID NOs: 13 and 14, respectively; or (3) SEQ ID NOs: 17 and 18, respectively.

6. The isolated monoclonal antibody or the antigen-binding portion thereof according to any one of claims 1 to 5, **characterised in that** the antibody or the antigen-binding portion thereof further comprises a heavy chain constant region and/or a light chain constant region.

7. The isolated monoclonal antibody or the antigen-binding portion thereof according to claim 6, **characterised in that** the heavy chain constant region is an IgG1 heavy chain constant region.

8. The isolated monoclonal antibody or the antigen-binding portion thereof according to claim 7, **characterised in that** the heavy chain constant region is a human IgG1 heavy chain constant region comprising an amino acid sequence set forth in Uniprot Accession No. P01857-1.

9. The isolated monoclonal antibody or the antigen-binding portion thereof according to claim 6, **characterised in that** the light chain constant region is a κ light chain constant region.

10. The isolated monoclonal antibody or the antigen-binding portion thereof according to claim 9, **characterised in that** the light chain constant region is a human κ light chain constant region comprising an amino acid sequence set forth in Uniprot Accession No. P01834.

11. The isolated monoclonal antibody or the antigen-binding portion thereof according to any one of claims 1 to 10, **characterised in that** the antibody or the antigen-binding portion thereof is human or chimeric.

12. The isolated monoclonal antibody or the antigen-binding portion thereof according to any one of claims 1 to 11, **characterised in that** the antibody or the antigen-binding portion thereof i) is capable of binding to human PD-L1, ii) is capable of binding to monkey PD-L1, iii) is capable of binding to mouse PD-L1, iv) blocks the binding of PD-1 to PD-L1, and/or v) exhibits an *in vivo* anti-tumor effect.

13. An immunoconjugate, **characterised in that** the immunoconjugate comprises i) the antibody or the antigen-binding portion thereof according to any one of claims 1 to 12; and ii) a cytotoxic molecule, wherein i) is linked to ii) via a linker or directly linked.

14. A bispecific molecule, **characterised in that** the bispecific molecule comprises: i) the antibody or the antigen-binding portion thereof according to any one of claims 1 to 12; and ii) a second functional moiety, wherein i) and ii) bind to different antigens or different epitopes of the same antigen, and i) is linked to ii).

15. A chimeric antigen receptor, **characterised in that** the chimeric antigen receptor comprises: i) an extracellular antigen-binding domain, ii) a transmembrane domain, and iii) an intracellular signaling domain, wherein the extracellular antigen-binding domain comprises the antibody or the antigen-binding portion thereof according to any one of claims 1 to 12.

16. An immune cell, **characterised in that** the immune cell comprises the chimeric antigen receptor according to claim 15.

17. An oncolytic virus, **characterised in that** the oncolytic virus expresses the antibody or the antigen-binding portion thereof according to any one of claims 1 to 12.

18. A nucleic acid molecule, **characterised in that** the nucleic acid molecule encodes the antibody or the antigen-binding portion thereof according to any one of claims 1 to 12, the immunoconjugate according to claim 13, the bispecific molecule according to claim 14, or the chimeric antigen receptor according to claim 15.

19. An expression vector, **characterised in that** the expression vector comprises the nucleic acid molecule according to claim 18.

20. A host cell, **characterised in that** the host cell comprises the expression vector according to claim 19, or has the nucleic acid molecule according to claim 18 integrated into the genome thereof.

21. A composition, **characterised in that** the composition comprises the antibody or the antigen-binding portion thereof according to any one of claims 1 to 12, the immunoconjugate according to claim 13, the bispecific molecule according to claim 14, the chimeric antigen receptor according to claim 15, the immune cell according to claim 16, the oncolytic virus according to claim 17, the nucleic acid molecule according to claim 18, the expression vector according to claim 19, or the host cell according to claim 20.

22. The composition according to claim 21, **characterised in that** the composition is a pharmaceutical composition, and further comprises a pharmaceutically acceptable carrier.

23. Use of the composition according to claim 21 or 22 in the preparation of a drug for treating or alleviating a PD-L1-associated cancer.

24. The use according to claim 23, **characterised in that** the PD-L1-associated cancer is a solid tumor.

25. The use according to claim 24, **characterised in that** the solid tumor is melanoma, non-small cell lung cancer, renal cell carcinoma, bladder cancer, or head and neck squamous cell carcinoma.
